# EUROPEAN PATENT SPECIFICATION

(11) **EP 3 207 491 B1**
(45) Date of publication and mention of the grant of the patent: **08.04.2020**
(21) Application number: 15793883.8
(22) Date of filing: 12.10.2015
(51) Int. Cl.: G06K 7/10, A61B 90/98, A61B 90/90, A61B 90/00

(54) **SURGICAL INSTRUMENT RFID TAG READING APPARATUS AND METHOD, AND SURGICAL INSTRUMENT TRACKING SYSTEM**
RFID-ETIKETT-LESEVORRICHTUNG FÜR CHIRURGISCHES INSTRUMENT UND VERFAHREN SOWIE VERFOLGUNGSSYSTEM FÜR CHIRURGISCHES INSTRUMENT
APPAREIL ET PROCÉDÉ DE LECTURE D'ÉTIQUETTE RFID D'INSTRUMENT CHIRURGICAL, ET SYSTÈME DE SUIVI D'INSTRUMENT CHIRURGICAL

(30) Priority: 15.10.2014 GB 201418293
(43) Date of publication of application: 23.08.2017
(73) Proprietor: SPA Track Medical Limited, Droitwich Spa, Worcestershire WR9 0QG (GB)
(72) Inventor: JOHNSON, Martin Stanley, Droitwich Spa Worcestershire WR9 0RG (GB); LYNCH, Mark Howard, Droitwich Spa Worcestershire WR9 0QG (GB); O'DONNELL, Andrew, Holmfirth Yorkshire HD9 3UA (GB); CALLAGHAN, Paul Dominic, Huddersfield Yorkshire HD7 5HA (GB)
(74) Representative: Molony, Anna
(86) International application number: PCT/GB2015/052977
(87) International publication number: WO 2016/059382

(56) References cited:
- WO-A1-2013/104743
- DE-A1-102012 107 274
- US-A1- 2006 043 177
- US-A1- 2011 112 384

## Description

### Technical Field

The invention relates to surgical instrument radio frequency identification, RFID, tag reading apparatus. The invention further relates to a surgical instrument tracking system. The invention further relates to a method of reading RFID tags on a plurality of surgical instruments of a surgical instrument set, each RFID tag containing identification information for the respective surgical instrument.

### Background

A surgeon typically requires a number of specific sets of surgical instruments in order to perform an operation. Before the operation can be started a check of each instrument set must be made to confirm that all the necessary surgical instruments are present. This process is typically repeated once the operation has been carried out, both before and after the patient is closed up. Each of these checks is typically carried out by two members of the surgical team.

Reusable surgical instruments are required to be decontaminated and sterilised before they can be used again. Surgical instrument sets are typically provided within trays, and during the decontamination and sterilisation process the surgical instruments in each set typically remain together. The instruments in each set are checked off against an instrument list at least twice during the decontamination and sterilisation process; once on the dirty side of the process, before the process is started and once on the clean side of the process, after the process has been completed. Historically the decontamination and sterilisation process has been carried out by the central sterile services departments, CSSD, of the hospitals in which the instruments are used but economic pressures are driving some hospitals to outsource their CSSD functions to offsite, third party providers. Hospitals are increasingly being required to track each surgical instrument over a period of years in order to manage contamination risk, especially Creutzfeldt-Jakob disease, CJD. The correct identification of each surgical instrument, and its originating hospital, during the decontamination and sterilisation process is therefore becoming increasingly important.

Rather than relying on visual identification by highly trained staff of each of hundreds of different types of surgical instrument, various labelling systems are available which are directly applied to each surgical instrument. These include etched bar codes, stick-on labels and RFID tags. The bar codes and labels suffer from deterioration due to the harsh conditions to which they are exposed during the decontamination and sterilisation process, and include edges which can provide locations for prions, such as CJD prions, to attach themselves to. RFID tags may offer a solution to these problems but current RFID tag readers for surgical instruments can only read the RFID tags on a few surgical instruments at a time and require the instruments to be separated from their set during reading. For example, EP2218421 describes an identification antenna for RFID tags attached to surgical instruments which are placed on the antenna.

US 2006/043177 describes a radio frequency identification (RFID) reader device for interrogating a smart surgical instrument tray with a number of surgical instruments that are tagged with RFID read/write tags that identify each surgical instrument in terms of manufacturer, part number, name, usage, maintenance history. The the surgical instrument tray arrives at a distribution centre, via a conveyer belt on which the RFID reader is mounted. As the surgical instrument tray is presented into a wireless radio frequency field of the RFID reader device, an interrogation signal is emitted that activates the RFID tags that are placed on the surgical instruments, and enables a response by the RFID tags via a transceiver/antenna combination. The transceivers and the antenna collect data from the RFID tagged surgical instruments and pass the data to a data output device, such as a desk top PC.

US2011/112384 describes a medical system which comprises a central control unit for displaying a diagnostic measured value using a display element and an invasive unit, having an invasive consumable. The invasive consumable is set up to invasively intervene in a tissue of a patient. The invasive unit has a contactlessly readable electronic identifier for storing at least one piece of information. The central control unit is set up to electronically read the information in the electronic identifier.

WO2013/104743 describes a storage/transport container for medical instruments comprising a container for receiving medical instruments and a device for detecting and transferring data of medical instruments. The device comprises a control unit, a reading unit and an identification element arranged on a medical instrument. The control unit is connected to the reading unit and exchanges data with the reading unit and processes, stores and/or forwards the data to a second identification element on the container. The reading unit exchanges data with the identification element and forwards the data to the control unit. The device has a sensor unit which detects the medical instrument by measuring a variable and exchanges signals with the control unit, which initiate the exchange of data between the reading unit and the identification element, and the reading unit transfers the exchanged data to the control unit.

DE 10 2012 107 274 discloses a device for simultaneously identifying a plurality of surgical instruments including an RFID tag reader, a camera, a barcode reader and a weighing device. Vibrating means are provided to apply vibration to the instruments in order to move the instruments to allow instruments undetected by the camera or the barcode reader to be read during a second reading operation.

### Summary

The invention is defined in the appended independent claims. Preferred embodiments are disclosed in the dependent claims. It is an object to provide an improved surgical instrument radio frequency identification, RFID, tag reading apparatus. It is a further object to provide an improved surgical instrument tracking system. It is a further object to provide an improved method of reading RFID tags on a plurality of surgical instruments of a surgical instrument set, each RFID tag containing identification information for the respective surgical instrument.

A first aspect of the disclosure provides surgical instrument radio frequency identification, RFID, tag reading apparatus comprising an interrogation zone, an RFID tag reader, a radio frequency, RF, antenna, guidance apparatus and a controller. The interrogation zone is arranged to receive a surgical instrument set comprising a plurality of surgical instruments. Each surgical instrument comprises a respective RFID tag containing identification information for the surgical instrument. The RF antenna is coupled to the RFID tag reader and arranged to transmit an RF signal. The RF signal has a signal field at least part of which extends within the interrogation zone. The RF antenna is arranged to receive RF return signals from at least some of the RFID tags, each RF return signal containing the identification information of the respective RFID tag. The guidance apparatus is arranged to cause a predetermined relative movement between the RFID tags on the surgical instruments and the RF signal. The controller is arranged to perform step a. of receiving the surgical instrument identification information from the RFID tag reader for each RFID tag from which an RF return signal is received. The controller is arranged to perform step b. of comparing the received surgical instrument identification information with a surgical instrument list of the surgical instrument set. And the controller is arranged to perform step c. of determining whether surgical instrument identification information has been received for each of the surgical instruments on the surgical instrument list, and if not, generate an incomplete list alert. The guidance apparatus is arranged to perform rotation and the controller is further arranged to, in response to an incomplete list alert perform step d. of transmitting a control signal to the guidance apparatus comprising instructions arranged to cause a predetermined rotational relative movement between the RFID tags on the surgical instruments and the RF signal. The predetermined rotational relative movement is different to said predetermined relative movement. The controller is further arranged then to repeat steps a. to c., wherein the repeated step b. performs a cumulative comparison of the received surgical instrument identification information with the surgical instrument list.

The surgical instrument RFID tag reading apparatus may enable the RFID tags on the surgical instruments in a set to be read whilst retaining the surgical instruments together. The apparatus may enable the surgical instruments in a set to be checked against an instrument list for the set without any manual handling of the instruments. The surgical instrument RFID tag reading apparatus may enable the RFID tags on the surgical instruments in a set to be read in a single process. Because the controller compares surgical instrument identification information received from the RFID tags on the surgical instruments with the instrument list the apparatus may not generate a false positive identification of the presence of a surgical instrument. The apparatus may therefore perform a more accurate check of the surgical instruments in a set than is possible when performing a manual check in which surgical instruments are visually identified and manually checked off a manual list. The apparatus may avoid incorrect identification of a surgical instrument and may avoid the wrong instrument being checked off the instrument list.

By performing a different predetermined relative movement between the RFID tags and the RF signal the apparatus may receive RF return signals from the RFID tags on instruments for which no return RF signal was received during the initial predetermined relative movement. Performing two different predetermined relative movements between the RF signal and the RFID tags may increase the likelihood of the apparatus reading the RFID tags on all of the surgical instruments in the set. Performing a cumulative comparison when repeating step b. may ensure that only those surgical instruments for which identification information has not previously been received are considered. The apparatus is able to check instruments off the instrument list cumulatively so that it is not necessary to receive identification information for each surgical instrument in the set during one predetermined relative movement.

In an embodiment, the controller is further arranged to, after repeating step c., generate a fail alert if surgical instrument identification information has still not been cumulatively received for all of the surgical instruments on the surgical instrument list.

In an embodiment, the apparatus further comprises a fail inspection zone and the guidance apparatus is arranged to deliver the surgical instrument set into the fail inspection zone in response to a said fail alert. This may ensure that instrument sets having one or more instruments missing from the instrument list, which may be due to the instrument's RFID tag not being read or the instrument being missing, are set to one side where a manual inspection for the missing instrument or instruments can be carried out.

In an embodiment, the guidance apparatus is arranged to guide movement of the surgical instrument set through the RF signal along a predetermined path. The predetermined relative movement between the RF signal and the surgical instrument set, into, through and out of the RF signal, is thereby performed along a predetermined path.

In an embodiment, the guidance apparatus comprises one of a guide rail and conveyor apparatus. The guide rail and the conveyor apparatus are each arranged to guide movement of the surgical instrument set through the RF signal along the predetermined path.

In an embodiment, the predetermined relative movement comprises movement along the predetermined path in a first direction and the further predetermined relative movement comprises movement along the predetermined path in a second direction, opposite to the first direction.

In an embodiment, the guidance apparatus is arranged to guide movement of the surgical instrument set along the predetermined linear path at one of a plurality of predetermined speeds. This may enable the apparatus to use a different speed for the relative movement and for the further relative movement. This may also enable the apparatus to use a different speed for different instrument sets, which may for example enable a lower speed to be used for instrument sets comprising a large number of surgical instruments. This may increase the likelihood of receiving a return RF signal for all of the surgical instruments in the instrument set.

In an embodiment, the guidance apparatus is arranged to guide movement of the surgical instrument set along the predetermined path at a speed which varies along said path. The speed varies according to a predetermined speed profile. This may increase the likelihood of receiving a return RF signal for all of the surgical instruments in the instrument set.

In an embodiment, the speed varies according to one of a plurality of predetermined speed profiles. This may enable the speed profile to be selected according to the instrument set, which may increase the likelihood of receiving a return RF signal for all of the surgical instruments in the instrument set.

In an embodiment, the guidance apparatus is arranged to at least one of rotate, tilt and shake the surgical instrument set. A predetermined rotational or tilting relative movement may therefore be caused between the RF signal and the surgical instrument set. Shaking the surgical instrument set may cause the position of at least some of the surgical instruments to change. This may increase the likelihood of receiving a return RF signal for all of the surgical instruments in the instrument set.

In an embodiment, the guidance apparatus comprises an interrogation platform arranged to receive the surgical instrument set. The interrogation platform is movably mounted and arranged to cause at least one of rotation, tilting and shaking of the surgical instrument set.

In an embodiment, the guidance apparatus is arranged to cause the relative movement between the RFID tags on the surgical instruments and the RF signal by guiding movement of the surgical instrument set through the RF signal along a first predetermined path in a first direction. The guidance apparatus is arranged to cause the further relative movement between the RFID tags on the surgical instruments and the RF signal by one of: guiding movement of the surgical instrument set through the RF signal along the first predetermined path in a second direction, different to the first direction; guiding movement of the surgical instrument set through the RF signal along a second predetermined path different to the first predetermined path; rotating the surgical instrument set; and tilting the surgical instrument set.

In an embodiment, the guidance apparatus is arranged to shake the surgical instrument set prior to the further relative movement.

In an embodiment, the surgical instrument set comprises an instrument carrier configured to carry the surgical instruments. The interrogation zone is arranged to receive the instrument carrier carrying the surgical instruments and the guidance apparatus is arranged to receive the instrument carrier carrying the surgical instruments and is arranged to cause said predetermined relative movement between the instrument carrier, and thus the surgical instruments, and the RF signal.

In an embodiment, the instrument carrier is one of a metal tray and a plastic tray.

In an embodiment, the instrument carrier comprises a base which reflects RF signals and an instrument platform which is at least partially transparent to RF signals. The instrument platform is mounted within the instrument carrier raised above the base. RF signals from the RF antenna may therefore be reflected from the base back towards the surgical instruments and RF return signals transmitted towards the base may be reflected back towards the RF antenna. This may increase the likelihood of receiving a return RF signal for all of the surgical instruments in the instrument set.

In an embodiment, the guidance apparatus comprises weighing apparatus. The controller is further arranged to receive a weight of the surgical instrument set from the weighing apparatus. The controller is arranged to compare the weight with a reference weight associated with the surgical instrument list. The controller is arranged to determine whether the received weight is substantially equal to the reference weight, and if not, generate an incomplete list alert. Comparing the received weight with the reference weight may provide a supplementary check of whether all of the surgical instruments on the instrument list are present.

In an embodiment, the guidance apparatus comprises an antenna carrier on which the RF antenna is mounted. The antenna carrier is movably mounted and arranged to undergo at least one of a predetermined linear movement, rotation and tilting such that at least one of a predetermined linear, rotational and tilting relative movement is caused between the RF signal and the surgical instrument set. A predetermined relative movement between the RF signal and the surgical instruments may therefore be achieved without movement of the surgical instruments. This may enable the apparatus to have a compact form. The apparatus may also perform movement of the RF signal in addition to movement of the surgical instruments, which may increase the likelihood of receiving a return RF signal for all of the surgical instruments in the instrument set.

In an embodiment, the apparatus comprises a second RF antenna coupled to an RFID tag reader. The RF antennas are arranged in a spaced relationship generally opposing each other such that the interrogation zone is located generally between the RF antennas. This may increase the likelihood of receiving a return RF signal for all of the surgical instruments in the instrument set.

In an embodiment, each RF antenna is coupled to the RFID tag reader. In an embodiment, each RF antenna is coupled to a respective RFID tag reader and the controller is arranged to receive surgical instrument identification information from each RFID tag reader.

A second aspect of the disclosure provides a surgical instrument RFID tag reading system comprising surgical instrument RFID tag reading apparatus and an instrument carrier. The instrument carrier comprises a base and an instrument platform. The base is reflective to RF signals. The instrument platform is at least partially transparent to RF signals and the instrument platform is mounted within the instrument carrier raised above the base. The RFID, tag reading apparatus comprises an interrogation zone, an RFID tag reader, a radio frequency, RF, antenna, guidance apparatus and a controller. The interrogation zone is arranged to receive a surgical instrument set comprising a plurality of surgical instruments. Each surgical instrument comprises a respective RFID tag containing identification information for the surgical instrument. The RF antenna is coupled to the RFID tag reader and arranged to transmit an RF signal. The RF signal has a signal field at least part of which extends within the interrogation zone. The RF antenna is arranged to receive RF return signals from at least some of the RFID tags, each return RF signal containing the identification information of the respective RFID tag. The guidance apparatus is arranged to cause relative movement between the RFID tags on the surgical instruments and the RF signal. The controller is arranged to perform step a. of receiving the surgical instrument identification information from the RFID tag reader for each RFID tag from which an RF return signal is received. The controller is arranged to perform step b. of comparing the received surgical instrument identification information with a surgical instrument list of the surgical instrument set. And the controller is arranged to perform step c. of determining whether surgical instrument identification information has been received for each of the surgical instruments on the surgical instrument list, and if not, generate an incomplete list alert. The guidance apparatus is arranged to perform rotation and the controller is further arranged to, in response to an incomplete list alert perform step d. of transmitting a control signal to the guidance apparatus comprising instructions arranged to cause a predetermined rotational relative movement between the RFID tags on the surgical instruments and the RF signal. The predetermined rotational relative movement is different to said predetermined relative movement. The controller is further arranged then to repeat steps a. to c., wherein the repeated step b. performs a cumulative comparison of the received surgical instrument identification information with the surgical instrument list.

The surgical instrument RFID tag reading system may enable the RFID tags on the surgical instruments in a set to be read whilst retaining the surgical instruments together. The raised instrument platform may enable RF signals from the RF antenna to be reflected from the base back towards the surgical instruments and RF return signals transmitted towards the base may be reflected back towards the RF antenna. This may increase the likelihood of receiving a return RF signal for all of the surgical instruments in the instrument set.

The system may enable the surgical instruments in a set to be checked against an instrument list for the set without any manual handling of the instruments. The surgical instrument RFID tag reading system may enable the RFID tags on the surgical instruments in a set to be read in a single process. Because the controller compares surgical instrument identification information received from the RFID tags on the surgical instruments with the instrument list the apparatus may not generate a false positive identification of the presence of a surgical instrument. The system may therefore perform a more accurate check of the surgical instruments in a set than is possible when performing a manual check in which surgical instruments are visually identified and manually checked off a manual list. The system may avoid incorrect identification of a surgical instrument and may avoid the wrong instrument being checked off the instrument list.

By performing a different predetermined relative movement between the RFID tags and the RF signal the apparatus may receive RF return signals from the RFID tags on instruments for which no return RF signal was received during the initial predetermined relative movement. Performing two different predetermined relative movements between the RF signal and the RFID tags may increase the likelihood of the apparatus reading the RFID tags on all of the surgical instruments in the set. Performing a cumulative comparison when repeating step b. may ensure that only those surgical instruments for which identification information has not previously been received are considered. The apparatus is able to check instruments off the instrument list cumulatively so that it is not necessary to receive identification information for each surgical instrument in the set during one predetermined relative movement.

In an embodiment, the controller is further arranged to, after repeating step c., generate a fail alert if surgical instrument identification information has still not been cumulatively received for all of the surgical instruments on the surgical instrument list.

In an embodiment, the apparatus further comprises a fail inspection zone and the guidance apparatus is arranged to deliver the surgical instrument set into the fail inspection zone in response to a said fail alert. This may ensure that instrument sets having one or more instruments missing from the instrument list, which may be due to the instrument's RFID tag not being read or the instrument being missing, are set to one side where a manual inspection for the missing instrument or instruments can be carried out.

In an embodiment, the guidance apparatus is arranged to guide movement of the surgical instrument set through the RF signal along a predetermined path. The predetermined relative movement between the RF signal and the surgical instrument set, into, through and out of the RF signal, is thereby performed along a predetermined path.

In an embodiment, the guidance apparatus comprises one of a guide rail and conveyor apparatus. The guide rail and the conveyor apparatus are each arranged to guide movement of the surgical instrument set through the RF signal along the predetermined path.

In an embodiment, the predetermined relative movement comprises movement along the predetermined path in a first direction and the further predetermined relative movement comprises movement along the predetermined path in a second direction, opposite to the first direction.

In an embodiment, the guidance apparatus is arranged to guide movement of the surgical instrument set along the predetermined linear path at one of a plurality of predetermined speeds. This may enable the apparatus to use a different speed for the relative movement and for the further relative movement. This may also enable the apparatus to use a different speed for different instrument sets, which may for example enable a lower speed to be used for instrument sets comprising a large number of surgical instruments. This may increase the likelihood of receiving a return RF signal for all of the surgical instruments in the instrument set.

In an embodiment, the guidance apparatus is arranged to guide movement of the surgical instrument set along the predetermined path at a speed which varies along said path. The speed varies according to a predetermined speed profile. This may increase the likelihood of receiving a return RF signal for all of the surgical instruments in the instrument set.

In an embodiment, the speed varies according to one of a plurality of predetermined speed profiles. This may enable the speed profile to be selected according to the instrument set, which may increase the likelihood of receiving a return RF signal for all of the surgical instruments in the instrument set.

In an embodiment, the guidance apparatus is arranged to at least one of rotate, tilt and shake the surgical instrument set. A predetermined rotational or tilting relative movement may therefore be caused between the RF signal and the surgical instrument set. Shaking the surgical instrument set may cause the position of at least some of the surgical instruments to change. This may increase the likelihood of receiving a return RF signal for all of the surgical instruments in the instrument set.

In an embodiment, the guidance apparatus comprises an interrogation platform arranged to receive the surgical instrument set. The interrogation platform is movably mounted and arranged to cause at least one of rotation, tilting and shaking of the surgical instrument set.

In an embodiment, the guidance apparatus is arranged to cause the relative movement between the RFID tags on the surgical instruments and the RF signal by guiding movement of the surgical instrument set through the RF signal along a first predetermined path in a first direction. The guidance apparatus is arranged to cause the further relative movement between the RFID tags on the surgical instruments and the RF signal by one of: guiding movement of the surgical instrument set through the RF signal along the first predetermined path in a second direction, different to the first direction; guiding movement of the surgical instrument set through the RF signal along a second predetermined path different to the first predetermined path; rotating the surgical instrument set; and tilting the surgical instrument set.

In an embodiment, the guidance apparatus is arranged to shake the surgical instrument set prior to the further relative movement.

In an embodiment, the surgical instrument set comprises an instrument carrier configured to carry the surgical instruments. The interrogation zone is arranged to receive the instrument carrier carrying the surgical instruments and the guidance apparatus is arranged to receive the instrument carrier carrying the surgical instruments and is arranged to cause said predetermined relative movement between the instrument carrier, and thus the surgical instruments, and the RF signal.

In an embodiment, the instrument carrier is one of a metal tray and a plastic tray.

In an embodiment, the instrument carrier comprises a base which reflects RF signals and an instrument platform which is at least partially transparent to RF signals. The instrument platform is mounted within the instrument carrier raised above the base. RF signals from the RF antenna may therefore be reflected from the base back towards the surgical instruments and RF return signals transmitted towards the base may be reflected back towards the RF antenna. This may increase the likelihood of receiving a return RF signal for all of the surgical instruments in the instrument set.

In an embodiment, the guidance apparatus comprises weighing apparatus. The controller is further arranged to receive a weight of the surgical instrument set from the weighing apparatus. The controller is arranged to compare the weight with a reference weight associated with the surgical instrument list. The controller is arranged to determine whether the received weight is substantially equal to the reference weight, and if not, generate an incomplete list alert. Comparing the received weight with the reference weight may provide a supplementary check of whether all of the surgical instruments on the instrument list are present.

In an embodiment, the guidance apparatus comprises an antenna carrier on which the RF antenna is mounted. The antenna carrier is movably mounted and arranged to undergo at least one of a predetermined linear movement, rotation and tilting such that at least one of a predetermined linear, rotational and tilting relative movement is caused between the RF signal and the surgical instrument set. A predetermined relative movement between the RF signal and the surgical instruments may therefore be achieved without movement of the surgical instruments. This may enable the apparatus to have a compact form. The apparatus may also perform movement of the RF signal in addition to movement of the surgical instruments, which may increase the likelihood of receiving a return RF signal for all of the surgical instruments in the instrument set.

In an embodiment, the apparatus comprises a second RF antenna coupled to an RFID tag reader. The RF antennas are arranged in a spaced relationship generally opposing each other such that the interrogation zone is located generally between the RF antennas. This may increase the likelihood of receiving a return RF signal for all of the surgical instruments in the instrument set.

In an embodiment, each RF antenna is coupled to the RFID tag reader. In an embodiment, each RF antenna is coupled to a respective RFID tag reader and the controller is arranged to receive surgical instrument identification information from each RFID tag reader.

A third aspect of the disclosure provides a surgical instrument tracking system comprising a surgical instrument set and a plurality of surgical instrument RFID tag reading apparatus. The surgical instrument set comprises a plurality of surgical instruments each comprising a respective RFID tag containing identification information for the surgical instrument. Each surgical instrument RFID tag reading apparatus is arranged to receive the surgical instrument set. Each surgical instrument RFID reading apparatus comprises an interrogation zone, an RFID tag reader, a radio frequency, RF, antenna, guidance apparatus and a controller. The interrogation zone is arranged to receive the surgical instrument set. The RF antenna is coupled to the RFID tag reader and arranged to transmit an RF signal. The RF signal has a signal field at least part of which extends within the interrogation zone. The RF antenna is arranged to receive RF return signals from at least some of the RFID tags, each RF return signal containing the identification information of the respective RFID tag. The guidance apparatus is arranged to cause a predetermined relative movement between the RFID tags on the surgical instruments and the RF signal. The controller is arranged to perform step a. of receiving the surgical instrument identification information from the RFID tag reader for each RFID tag from which an RF return signal is received. The controller is arranged to perform step b. of comparing the received surgical instrument identification information with a surgical instrument list of the surgical instrument set. And the controller is arranged to perform step c. of determining whether surgical instrument identification information has been received for each of the surgical instruments on the surgical instrument list, and if not, generate an incomplete list alert. The controller is further arranged to, in response to an incomplete list alert perform step d. of transmitting a control signal to the guidance apparatus comprising instructions arranged to cause a further predetermined relative movement between the RFID tags on the surgical instruments and the RF signal. The further predetermined relative movement is different to said predetermined relative movement. The controller is further arranged then to repeat steps a. to c., wherein the repeated step b. performs a cumulative comparison of the received surgical instrument identification information with the surgical instrument list.

The surgical instrument tracking system may enable the RFID tags on the surgical instruments in a set to be read at a plurality of locations, whilst retaining the surgical instruments together. The system may enable the surgical instruments in a set to be checked at each location against an instrument list for the set without any manual handling of the instruments. The surgical instrument RFID tag reading apparatus may enable the RFID tags on the surgical instruments in a set to be read in a single process at each location. Because the controller compares surgical instrument identification information received from the RFID tags on the surgical instruments with the instrument list the system may not generate a false positive identification of the presence of a surgical instrument. The system may therefore perform more accurate checking and tracking of the surgical instruments in a set than is possible when performing manual checks in which surgical instruments are visually identified and manually checked off a manual list at each location. The surgical instrument RFID tag reading apparatus may avoid incorrect identification of a surgical instrument and may avoid the wrong instrument being checked off the instrument list.

By performing a different predetermined relative movement between the RFID tags and the RF signal the apparatus may receive RF return signals from the RFID tags on instruments for which no return RF signal was received during the initial predetermined relative movement. Performing two different predetermined relative movements between the RF signal and the RFID tags may increase the likelihood of the apparatus reading the RFID tags on all of the surgical instruments in the set. Performing a cumulative comparison when repeating step b. may ensure that only those surgical instruments for which identification information has not previously been received are considered. The apparatus is able to check instruments off the instrument list cumulatively so that it is not necessary to receive identification information for each surgical instrument in the set during one predetermined relative movement.

In an embodiment, the controller is further arranged to, after repeating step c., generate a fail alert if surgical instrument identification information has still not been cumulatively received for all of the surgical instruments on the surgical instrument list.

In an embodiment, the apparatus further comprises a fail inspection zone and the guidance apparatus is arranged to deliver the surgical instrument set into the fail inspection zone in response to a said fail alert. This may ensure that instrument sets having one or more instruments missing from the instrument list, which may be due to the instrument's RFID tag not being read or the instrument being missing, are set to one side where a manual inspection for the missing instrument or instruments can be carried out.

In an embodiment, the guidance apparatus is arranged to guide movement of the surgical instrument set through the RF signal along a predetermined path. The predetermined relative movement between the RF signal and the surgical instrument set, into, through and out of the RF signal, is thereby performed along a predetermined path.

In an embodiment, the guidance apparatus comprises one of a guide rail and conveyor apparatus. The guide rail and the conveyor apparatus are each arranged to guide movement of the surgical instrument set through the RF signal along the predetermined path.

In an embodiment, the predetermined relative movement comprises movement along the predetermined path in a first direction and the further predetermined relative movement comprises movement along the predetermined path in a second direction, opposite to the first direction.

In an embodiment, the guidance apparatus is arranged to guide movement of the surgical instrument set along the predetermined linear path at one of a plurality of predetermined speeds. This may enable the apparatus to use a different speed for the relative movement and for the further relative movement. This may also enable the apparatus to use a different speed for different instrument sets, which may for example enable a lower speed to be used for instrument sets comprising a large number of surgical instruments. This may increase the likelihood of receiving a return RF signal for all of the surgical instruments in the instrument set.

In an embodiment, the guidance apparatus is arranged to guide movement of the surgical instrument set along the predetermined path at a speed which varies along said path. The speed varies according to a predetermined speed profile. This may increase the likelihood of receiving a return RF signal for all of the surgical instruments in the instrument set.

In an embodiment, the speed varies according to one of a plurality of predetermined speed profiles. This may enable the speed profile to be selected according to the instrument set, which may increase the likelihood of receiving a return RF signal for all of the surgical instruments in the instrument set.

In an embodiment, the guidance apparatus is arranged to at least one of rotate, tilt and shake the surgical instrument set. A predetermined rotational or tilting relative movement may therefore be caused between the RF signal and the surgical instrument set. Shaking the surgical instrument set may cause the position of at least some of the surgical instruments to change. This may increase the likelihood of receiving a return RF signal for all of the surgical instruments in the instrument set.

In an embodiment, the guidance apparatus comprises an interrogation platform arranged to receive the surgical instrument set. The interrogation platform is movably mounted and arranged to cause at least one of rotation, tilting and shaking of the surgical instrument set.

In an embodiment, the guidance apparatus is arranged to cause the relative movement between the RFID tags on the surgical instruments and the RF signal by guiding movement of the surgical instrument set through the RF signal along a first predetermined path in a first direction. The guidance apparatus is arranged to cause the further relative movement between the RFID tags on the surgical instruments and the RF signal by one of: guiding movement of the surgical instrument set through the RF signal along the first predetermined path in a second direction, different to the first direction; guiding movement of the surgical instrument set through the RF signal along a second predetermined path different to the first predetermined path; rotating the surgical instrument set; and tilting the surgical instrument set.

In an embodiment, the guidance apparatus is arranged to shake the surgical instrument set prior to the further relative movement.

In an embodiment, the surgical instrument set comprises an instrument carrier configured to carry the surgical instruments. The interrogation zone is arranged to receive the instrument carrier carrying the surgical instruments and the guidance apparatus is arranged to receive the instrument carrier carrying the surgical instruments and is arranged to cause said predetermined relative movement between the instrument carrier, and thus the surgical instruments, and the RF signal.

In an embodiment, the instrument carrier is one of a metal tray and a plastic tray.

In an embodiment, the instrument carrier comprises a base which reflects RF signals and an instrument platform which is at least partially transparent to RF signals. The instrument platform is mounted within the instrument carrier raised above the base. RF signals from the RF antenna may therefore be reflected from the base back towards the surgical instruments and RF return signals transmitted towards the base may be reflected back towards the RF antenna. This may increase the likelihood of receiving a return RF signal for all of the surgical instruments in the instrument set.

In an embodiment, the guidance apparatus comprises weighing apparatus. The controller is further arranged to receive a weight of the surgical instrument set from the weighing apparatus. The controller is arranged to compare the weight with a reference weight associated with the surgical instrument list. The controller is arranged to determine whether the received weight is substantially equal to the reference weight, and if not, generate an incomplete list alert. Comparing the received weight with the reference weight may provide a supplementary check of whether all of the surgical instruments on the instrument list are present.

In an embodiment, the guidance apparatus comprises an antenna carrier on which the RF antenna is mounted. The antenna carrier is movably mounted and arranged to undergo at least one of linear movement, rotation and tilting such that at least one of a predetermined linear, rotational and tilting relative movement is caused between the RF signal and the surgical instrument set. A predetermined relative movement between the RF signal and the surgical instruments may therefore be achieved without movement of the surgical instruments. This may enable the apparatus to have a compact form. The apparatus may also perform movement of the RF signal in addition to movement of the surgical instruments, which may increase the likelihood of receiving a return RF signal for all of the surgical instruments in the instrument set.

In an embodiment, the apparatus comprises a second RF antenna coupled to an RFID tag reader. The RF antennas are arranged in a spaced relationship generally opposing each other such that the interrogation zone is located generally between the RF antennas. This may increase the likelihood of receiving a return RF signal for all of the surgical instruments in the instrument set.

In an embodiment, each RF antenna is coupled to the RFID tag reader. In an embodiment, each RF antenna is coupled to a respective RFID tag reader and the controller is arranged to receive surgical instrument identification information from each RFID tag reader.

A fourth aspect of the disclosure provides a method of reading RFID tags on a plurality of surgical instruments of a surgical instrument set. Each RFID tag contains identification information for the respective surgical instrument. The method comprises step a. of transmitting an RF signal having a signal field. The method comprises step b. of locating the RFID tags within the RF signal field. The method comprises step c. of causing a predetermined relative movement between the RF signal and the RFID tags. The method comprises step d. of receiving a RF return signal from at least some of the RFID tags. Each RF return signal contains the identification information of the respective RFID tag. The method comprises step e. of obtaining the surgical instrument identification information from each RF return signal. The method comprises step f. of comparing the received surgical instrument identification information with a surgical instrument list of the surgical instrument set. The method comprises step g. of determining whether surgical instrument identification information has been received for each of the surgical instruments on the surgical instrument list. If surgical instrument identification information has been received for each of the surgical instruments on the surgical instrument list, an incomplete list alert is generated. The method further comprises, in response to an incomplete list alert, step h. of causing a further predetermined relative movement between the RF signal and the RFID tags. The predetermined further relative movement is different to said predetermined relative movement. The method then repeats steps a. to g. By performing a different predetermined relative movement between the RFID tags and the RF signal, RF return signals may be received from the RFID tags on instruments for which no return RF signal was received during the initial relative movement.

The method may enable the RFID tags on the surgical instruments in a set to be read whilst retaining the surgical instruments together. The method may enable the surgical instruments in a set to be checked against an instrument list for the set without any manual handling of the instruments. The method may enable the RFID tags on the surgical instruments in a set to be read in a single process. Because the method compares surgical instrument identification information received from the RFID tags on the surgical instruments with the instrument list the method may not generate a false positive identification of the presence of a surgical instrument. The method may therefore perform a more accurate check of the surgical instruments in a set than is possible when performing a manual check in which surgical instruments are visually identified and manually checked off a manual list. The method may avoid incorrect identification of a surgical instrument and may avoid the wrong instrument being checked off the instrument list.

Performing two different predetermined relative movements between the RF signal and the RFID tags may increase the likelihood of reading the RFID tags on all of the surgical instruments in the set. Performing a cumulative comparison when repeating step f. may ensure that only those surgical instruments for which identification information has not previously been received are considered. The method is able to check instruments off the instrument list cumulatively so that it is not necessary to receive identification information for each surgical instrument in the set during one relative movement.

In an embodiment, the method further comprises, after repeating step g., generating a fail alert if surgical instrument identification information has still not been cumulatively received for all of the surgical instruments on the surgical instrument list.

In an embodiment, the method further comprises delivering the surgical instrument set into a fail inspection zone in response to a said fail alert. This may ensure that instrument sets having one or more instruments missing from the instrument list, which may be due to the instrument's RFID tag not being read or the instrument being missing, are set to one side where a manual inspection for the missing instrument or instruments can be carried out.

In an embodiment, the predetermined relative movement comprises movement along a predetermined path.

In an embodiment, the predetermined relative movement comprises movement along the predetermined path at a speed which varies along said path, and wherein the speed is varied along said path according to a predetermined speed profile. This may enable a different speed profile to be used for the relative movement and for the further relative movement. This may also enable different speeds to be used for different instrument sets, which may for example enable a lower speed to be used for instrument sets comprising a large number of surgical instruments. This may increase the likelihood of receiving a return RF signal for all of the surgical instruments in the instrument set.

In an embodiment, in step h. the further relative movement is one of movement in a second direction, different to the first direction, rotation by a second angle different to the first angle, tilting in a second plane different to the first plane, tilting by a second angle different to the first angle, or a different one of linear movement in a first direction, rotation by a first angle, and tilting in a first plane and by a first angle. This may increase the likelihood of receiving a return RF signal for all of the surgical instruments in the instrument set.

In an embodiment, step h. additionally comprises shaking the surgical instrument set prior to the further relative movement such that the position of at least some of the surgical instruments is changed. This may increase the likelihood of receiving a return RF signal for all of the surgical instruments in the instrument set.

In an embodiment, the surgical instrument set comprises an instrument carrier configured to carry the surgical instruments. Step c. comprises causing relative movement between the RF signal and the instrument carrier, and thus the RFID tags.

In an embodiment, the relative movement between the RF signal and the RFID tags comprises movement of the surgical instruments through the RF signal.

In an embodiment, the further relative movement between the RF signal and the RFID tags comprises movement of the surgical instruments through the RF signal.

In an embodiment, the relative movement between the RF signal and the RFID tags comprises movement of the RF signal across the surgical instruments.

In an embodiment, the further relative movement between the RF signal and the RFID tags comprises movement of the RF signal across the surgical instruments.

A fifth aspect of the disclosure provides a computer program comprising instructions which, when executed on at least one processor, cause the at least one processor to perform any of the steps of the above method of reading RFID tags on a plurality of surgical instruments of a surgical instrument set.

A sixth aspect of the disclosure provides a carrier containing the computer program, wherein the carrier is one of an electronic signal, optical signal, radio signal, or computer readable storage medium.

Embodiments of the disclosure will now be described, by way of example only, with reference to the accompanying drawings.

### Brief Description of the drawings

Figure 1 is a diagrammatic representation of surgical instrument RFID tag reading apparatus according to a first embodiment;
Figure 2 is a diagrammatic representation of surgical instrument RFID tag reading apparatus according to a second embodiment;
Figure 3 is a diagrammatic representation of surgical instrument RFID tag reading apparatus according to a fourth embodiment;
Figure 4 is a diagrammatic representation of surgical instrument RFID tag reading apparatus according to a sixth embodiment;
Figure 5 is a diagrammatic representation of surgical instrument RFID tag reading apparatus according to a embodiment;
Figure 6 is a diagrammatic side view of surgical instrument RFID tag reading apparatus according to an eleventh embodiment;
Figure 7 is a diagrammatic end view of the surgical instrument RFID tag reading apparatus of Figure 6;
Figure 8 is a diagrammatic representation of surgical instrument RFID tag reading apparatus according to a twelfth embodiment;
Figure 9 is a diagrammatic representation of surgical instrument RFID tag reading apparatus according to a thirteenth embodiment;
Figure 10 is a diagrammatic representation of surgical instrument RFID tag reading apparatus according to a fourteenth embodiment;
Figure 11 is a diagrammatic representation of a surgical instrument tracking system according to a fifteenth embodiment;
Figure 12 is a diagrammatic representation of a surgical instrument tracking system according to a sixteenth embodiment;
Figure 13 shows the steps of a method according to a seventeenth embodiment of reading RFID tags on a plurality of surgical instruments of a surgical instrument set;
Figure 14 shows the steps of a method according to a nineteenth embodiment of reading RFID tags on a plurality of surgical instruments of a surgical instrument set;
Figure 15 shows the steps of a method according to a twentieth embodiment of reading RFID tags on a plurality of surgical instruments of a surgical instrument set;
Figure 16 shows the steps of a method according to a twenty-first embodiment of reading RFID tags on a plurality of surgical instruments of a surgical instrument set;
Figure 17 shows the steps of a method according to a twenty-second embodiment of reading RFID tags on a plurality of surgical instruments of a surgical instrument set;
Figure 18 shows the steps of a method according to a twenty-third embodiment of reading RFID tags on a plurality of surgical instruments of a surgical instrument set;
Figure 19 is a diagrammatic representation of a surgical instrument RFID tag reading system according to a twenty-fourth embodiment; and
Figure 20 is a part cut-away view of the instrument tray of Figure 19.

### Detailed description

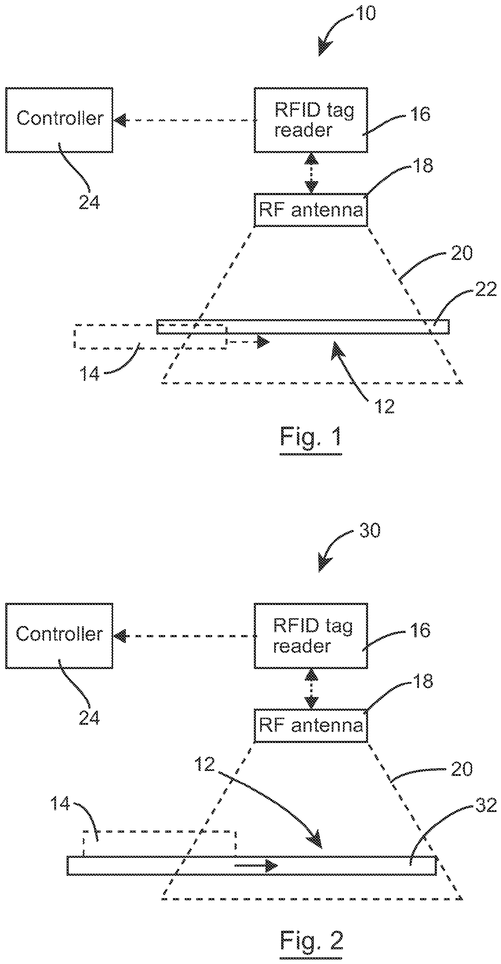

Referring to Figure 1, a first embodiment provides surgical instrument radio frequency identification, RFID, tag reading apparatus 10. The apparatus 10 comprises an interrogation zone 12, an RFID tag reader 14, a radio frequency, RF, antenna 18, guidance apparatus 22 and a controller 24.

The interrogation zone 12 is arranged to receive a surgical instrument set 14 (shown for clarity in Figure 1 but not forming part of this embodiment). The surgical instrument set comprises a plurality of surgical instruments, each of which has a respective RFID tag provided on it. Each RFID tag contains identification information for its surgical instrument.

The RF antenna 18 is coupled to the RFID tag reader 16. The RF antenna 18 is arranged to transmit an RF signal 20 having a signal field at least part of which extends within the interrogation zone. The RF signal field is also arranged to receive RF return signals from the RFID tags on the surgical instruments. Each RF return signal will contain the identification information of the respective RFID tag. RFID tags, RF antennas and RFID tag readers will be well known to the skilled person, so their structure, signalling and operation will not be described in further detail here.

The guidance apparatus of this embodiment takes the form of a guide rail 22 which is arranged such that the surgical instrument set 14 can move along the guide rail, in the direction indicated by the arrow, along a predetermined path. The movement of the surgical instrument set along the guide rail causes predetermined relative movement between the RFID tags on the surgical instruments and the RF signal 20.

The controller 24 is arranged to receive the surgical instrument identification information from the RFID tag reader for each of the RFID tags from which an RF return signal has been received. The controller is arranged to compare the received surgical instrument identification information with a surgical instrument list of the surgical instrument set. The surgical instrument list specifies each surgical instrument which should be present within the surgical instrument set. The controller is arranged to determine whether surgical instrument identification information has been received for each of the surgical instruments on the surgical instrument list. If surgical instrument identification information has not been received for each of the surgical instruments on the surgical instrument list, the controller generates an incomplete list alert. Missing surgical instrument identification information for a surgical instrument on the instrument list indicates that an RFID tag has not been read for that surgical instrument. This may be because the RFID tag did not see the RF signal, the RF antenna did not see the return RF signal transmitted by the RFID tag, or the surgical instrument is missing from the set. The apparatus 10 may therefore generate a false negative due to signalling error but it cannot generate a false positive, since a return RF signal will not be received if a surgical instrument RFID tag is not present.

A second embodiment provides surgical instrument radio frequency identification, RFID, tag reading apparatus 30 as shown in Figure 2. The apparatus 30 of this embodiment is similar to the apparatus 10 of Figure 1, with the following modifications. The same reference number are retained for corresponding features.

In this embodiment, the guidance apparatus takes the form of a conveyor 32, which may be a belt conveyor or a roller conveyor. The conveyor 32 is arranged to transport the surgical instrument set 14 into and through the RF signal in the direction of the arrow, along a predetermined path. It will be appreciated however that the conveyor 32 could equally be arranged to transport the surgical instrument set in the opposite direction.

A third embodiment provides surgical instrument radio frequency identification, RFID, tag reading apparatus having the same general structure as the apparatus shown in Figure 2. In this embodiment, the conveyor is arranged to guide movement of the surgical instrument set along the predetermined path at a speed which varies along the path. The speed varies according to a preselected speed profile. The speed profile may be a linearly increasing or decreasing speed or may be a nonlinear profile, for example with the speed increasing or decreasing along one or more sections of the path and remaining constant along one or more other sections.

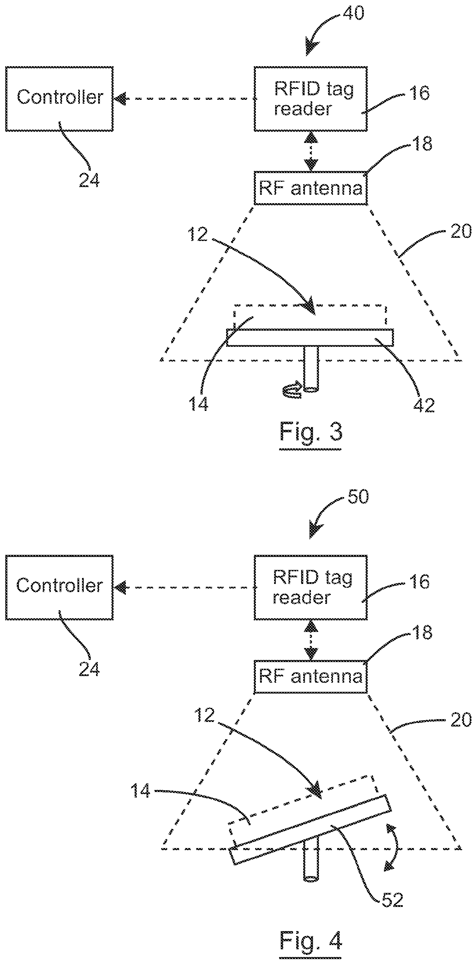

Figure 3 shows surgical instrument radio frequency identification, RFID, tag reading apparatus 40 according to a fourth embodiment. The apparatus of this embodiment is similar to the apparatus 10 of Figure 1, with the following modifications. The same reference number are retained for corresponding features.

In this embodiment, the guidance apparatus comprises an interrogation platform 42 which is arranged to receive the surgical instrument set 14. The guidance apparatus is movably mounted and is arranged to rotate, as indicated by the arrow, to rotate the surgical instrument set within the RF signal 20. This causes a predetermined rotational relative movement between the RF signal and the RFID tags on the surgical instruments in the surgical instrument set.

A fifth embodiment provides surgical instrument radio frequency identification, RFID, tag reading apparatus having the same general structure as the apparatus 40 shown in Figure 3. In this embodiment, the guidance apparatus additionally comprises weighing apparatus, which in this embodiment is incorporated into the interrogation platform 42. The weighing apparatus is arranged to generate an instrument set weight signal containing the weight measured by the weighing apparatus.

The controller is arranged to receive the weight of the surgical instrument set from the weighing apparatus and to compare the weight with a reference weight associated with the surgical instrument list. The controller is arranged to determine whether the received weight is substantially equal to the reference weight, indicating that all of the surgical instruments on the instrument list are present. It will be appreciated that the reference weight will in practice be a weight range, to allow for differences in the actual weight of each surgical instrument, within a known manufacturing tolerance. If the received weight is not equal to the reference weight, within the weight range, this indicates that one or more surgical instruments are missing, and the controller is arranged to generate an incomplete list alert when this occurs.

Figure 4 shows surgical instrument radio frequency identification, RFID, tag reading apparatus 50 according to a sixth embodiment. The apparatus of this embodiment is similar to the apparatus 10 of Figure 1, with the following modifications. The same reference number are retained for corresponding features.

In this embodiment, the guidance apparatus comprises an interrogation platform 52 which is arranged to receive the surgical instrument set 14. The guidance apparatus is movably mounted and is arranged to tilt, as indicated by the arrow, to tilt the surgical instrument set within the RF signal 20. This causes tilting relative movement between the RF signal and the RFID tags on the surgical instruments in the surgical instrument set.

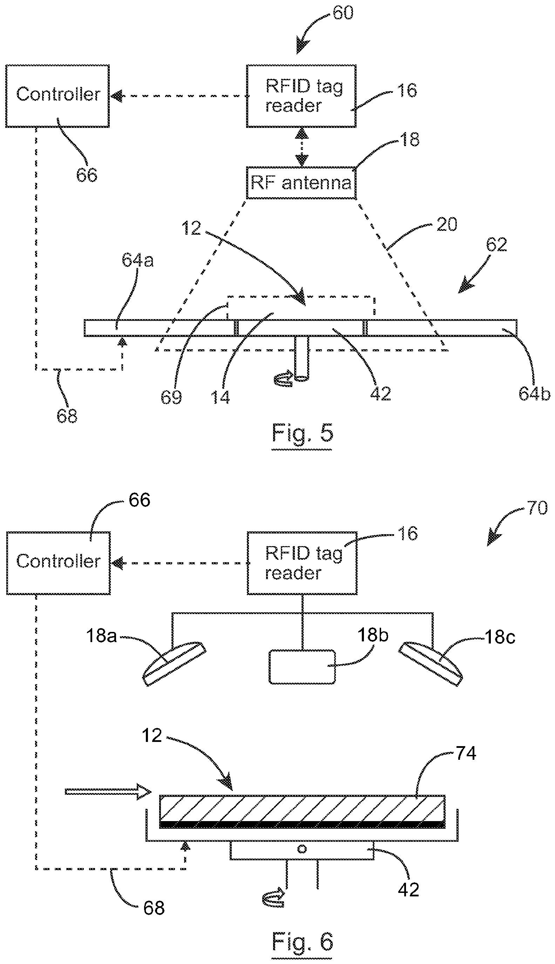

A seventh embodiment provides surgical instrument radio frequency identification, RFID, tag reading apparatus 60 as shown in Figure 5. The apparatus 60 of this embodiment is similar to the apparatus 30 of Figure 2 and the apparatus 40 of Figure 3, with the following modifications. The same reference number are retained for corresponding features.

In this embodiment, the guidance apparatus comprises both conveyor apparatus 62 and an interrogation platform 42. The surgical instrument set additionally comprises an instrument carrier, this example a metal instrument tray 69, in which the surgical instruments are carried. The interrogation zone is arranged to receive the instrument tray carrying the surgical instruments. The conveyors 64a, 64b are arranged to transport the instrument tray, and thus the surgical instruments into and through the RF signal. It will be appreciated that a plastic instrument tray may alternatively by used, and the instrument tray will typically of the type known as an autoclavable instrument tray.

The conveyor 62 comprises two conveyor belts 64a, 64b, provided on opposing sides of the interrogation platform. The interrogation platform 62 is movably mounted and is arranged to rotate, as indicated by the arrow, to rotate the surgical instrument set 14, 69 within the RF signal 20. It will be appreciated that the interrogation platform 52, arranged to tilt the surgical instrument set within the RF signal, may alternatively be used, as may an interrogation platform configured for both rotational and tilting movement.

Each conveyor belt 64a, 64b is arranged to transport the surgical instrument tray 69 along a predetermined path in two opposing linear directions, so that the surgical instrument set may be moved into, through and out of the RF signal 20 in either direction.

The controller 66 of this embodiment is arranged to transmit a first control signal 68 to the guidance apparatus 42, 63 comprising instructions arranged to cause an initial predetermined relative movement between the RFID tags on the surgical instruments and the RF signal. The controller 66 is additionally arranged, in response to an incomplete list alert, after the initial relative movement has been completed, to generate a second control signal 68 to the guidance apparatus 42, 63 comprising instructions arranged to cause further predetermined relative movement between the RFID tags on the surgical instruments and the RF signal. The further relative movement is different to the initial relative movement.

For example, the first control signal may comprise instructions arranged to cause the conveyor apparatus 62 to transport the surgical instrument tray 69 into the interrogation zone 12 and to deliver the surgical instrument tray onto the interrogation platform 42. If an incomplete list alert is generated, a second control signal is transmitted comprising instructions to cause the interrogation platform to rotate, thereby rotating the surgical instrument tray. It will be appreciated that when the interrogation platform 52, arranged to tilt, is used or an interrogation platform configured to both rotate and tilt, the second control signal will comprise instructions arranged to cause the interrogation platform to rotate, tilt or rotate and tilt.

An initial substantially linear predetermined relative movement between the RFID tags on the surgical instruments and the RF signal is thereby followed by a rotational and/or tilting predetermined relative movement between the RFID tags on the surgical instruments and the RF signal. The second control signal may, for example, cause the interrogation platform to rotate by 180° or 360°, or may cause the interrogation platform to rotate by +θ and then by -θ, to return the interrogation platform to its starting position. θ may be any angle up to 180°, although it is most preferably any angle up to 45°.

Alternatively, the first control signal may comprise instructions arranged to cause the conveyor apparatus to transport the surgical instrument set at a first speed, or with a first speed profile, and the second control signal may comprise instructions arranged to cause the conveyor apparatus to reverse direction and transport the surgical instrument set back through the RF signal in the opposite direction. This may be done at a second speed, or with a second speed profile, different to the first.

The following combinations of speed, reversing movement and rotation may, for example, be implemented:

| No. of passes through RF Signal | Speed | Rotation | Direction |
|---|---|---|---|
| 1 | Fast | nil | |
| 1 | Slow | nil | |
| 1 | Profile | nil | |
| | | | |
| 2 | 1 fast 2nd Fast | nil | |
| 2 | 1 Fast 2nd Slow | nil | |
| 2 | 1 Fast 2nd Profile | nil | |
| 2 | 1 Slow 2nd Slow | nil | |
| 2 | 1 Profile 2nd Profile | nil | |
| | | | |
| 1 | Fast | 180° | Clockwise |
| 1 | Slow | 180° | Clockwise |
| 1 | Profile | 180° | Clockwise |
| | | | |
| 2 | 1 fast 2nd Fast | 180° | Clockwise |
| 2 | 1 Fast 2nd Slow | 180° | Clockwise |
| 2 | 1 Fast 2nd Profile | 180° | Clockwise |
| 2 | 1 Slow 2nd Slow | 180° | Clockwise |
| 2 | 1 Profile 2nd Profile | 180° | Clockwise |
| 1 | Fast | 180° | Anticlockwise |
| 1 | Slow | 180° | Anticlockwise |
| 1 | Profile | 180° | Anticlockwise |
| | | | |
| 2 | 1 fast 2nd Fast | 180° | Anticlockwise |
| 2 | 1 Fast 2nd Slow | 180° | Anticlockwise |
| 2 | 1 Fast 2nd Profile | 180° | Anticlockwise |
| 2 | 1 Slow 2nd Slow | 180° | Anticlockwise |
| 2 | 1 Profile 2nd Profile | 180° | Anticlockwise |

As a result of the further predetermined relative movement, the controller again receives surgical instrument identification information. The controller is arranged to compare this surgical instrument identification information with the surgical instrument list and determine whether surgical instrument identification information has now been received for each of the surgical instruments on the surgical instrument list. The comparison is a cumulative comparison of the received surgical instrument identification information with the surgical instrument list. Performing a cumulative comparison when repeating the comparison means that only those surgical instruments for which identification information has not previously been received are considered the second time round. Because the instruments are checked off the instrument list cumulatively it is not necessary to receive identification information for each surgical instrument in the set during either relative movement alone.

If surgical instrument identification information has still not been received for each of the surgical instruments on the surgical instrument list, the controller generates a fail alert.

In an eighth embodiment, again described with reference to Figure 5, the controller is additionally arranged to generate a third control signal 68 comprising instructions to cause the conveyor apparatus 62 to move the surgical instrument tray 69 out of the RF signal, which may be done at a third speed or with a third speed profile. The apparatus 60 of this embodiment is therefore able to process a plurality of surgical instrument sets 14 in series, the surgical instrument trays 69 moving through the apparatus on the conveyor belts 64, one after another.

In a ninth embodiment, again described with reference to Figure 5, the second control signal 68 additionally comprises instructions to cause the conveyor apparatus 62 to move the surgical instrument tray 69 rapidly back and forth, thereby shaking the surgical instrument set. This causes the position of at least some of the surgical instruments to change within the tray prior to the further predetermined relative movement being performed.

In a tenth embodiment, again described with reference to Figure 5, the conveyors 64a, 64b are arranged to transport the surgical instrument set at a speed which varies along the path of the conveyors. The speed varies according to a preselected speed profile. The speed profile may be a linearly increasing or decreasing speed or may be a nonlinear profile, for example with the speed increasing or decreasing along one or more sections of the path and remaining constant along one or more other sections.

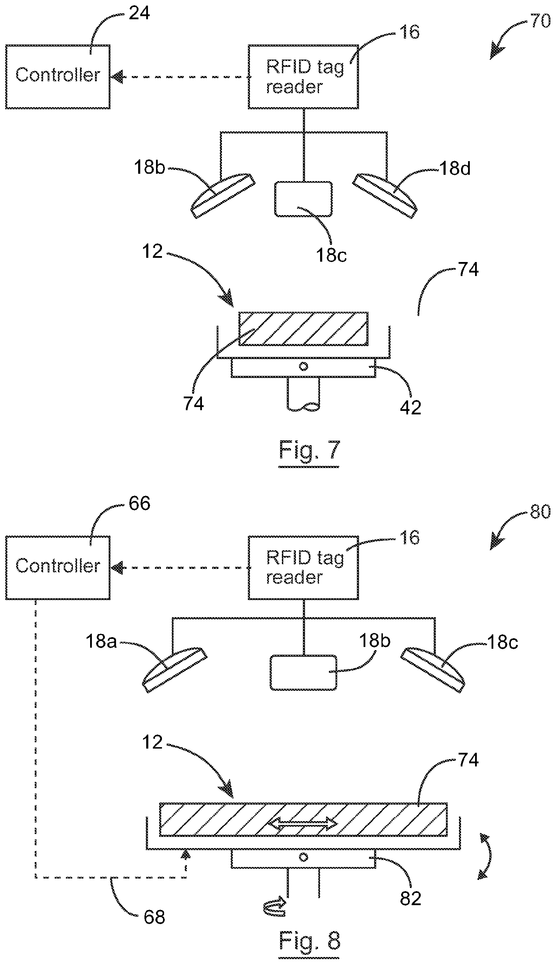

Figures 6 and 7 show surgical instrument radio frequency identification, RFID, tag reading apparatus 70 according to an eleventh embodiment. The apparatus of this embodiment is similar to the apparatus 60 of Figure 5, with the following modifications. The same reference number are retained for corresponding features.

In this embodiment, the apparatus 70 comprises four RF antennas 18a, 18b, 18c, 18d arranged as two opposing pairs 18a, 18c and 18b, 18d above the interrogation platform 42. Each of the antennas is coupled to the RFID tag reader 16. It will be appreciated that each antenna may alternatively be coupled to a respective RFID tag reader, and the controller 66 is arranged to receive surgical instrument identification information from each RFID tag reader.

The surgical instrument set 74 comprises a plurality of surgical instruments provided within an instrument tray, as described above. The surgical instrument tray is loaded onto the interrogation platform in the direction indicated by the arrow, thereby causing substantially linear relative movement between the RFID tags on the surgical instruments and the RF signal.

Figure 8 shows surgical instrument radio frequency identification, RFID, tag reading apparatus 80 according to a twelfth embodiment. The apparatus of this embodiment is similar to the apparatus 70 of Figures 6 and 7, with the following modifications. The same reference number are retained for corresponding features.

In this embodiment, the interrogation platform 82 is movably mounted and is arranged to rotate, tilt and shake, as indicated by the arrows, to rotate and tilt the surgical instrument set 74 within the RF signal 20, and the shake the surgical instruments within the tray.

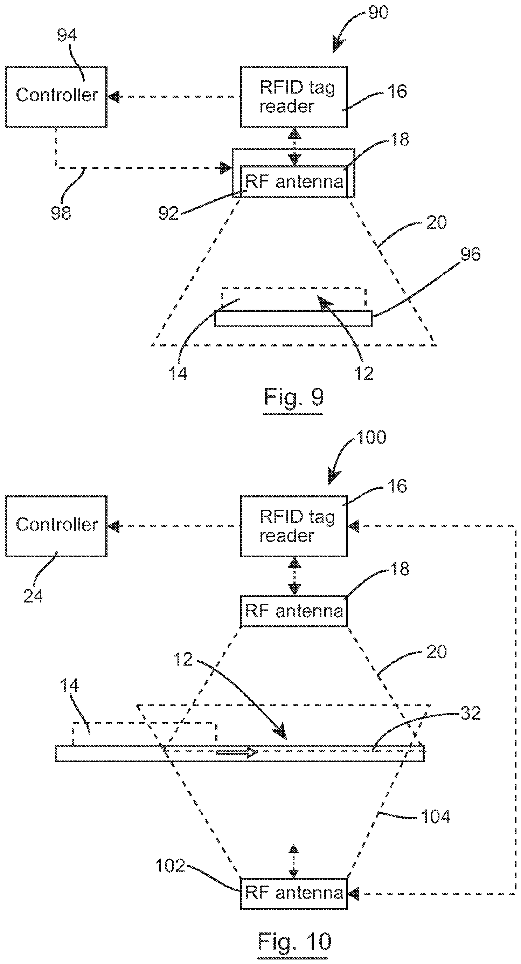

A thirteenth embodiment provides surgical instrument radio frequency identification, RFID, tag reading apparatus 90 as shown in Figure 9. The apparatus 90 of this embodiment is similar to the apparatus 10 of Figure 1, with the following modifications. The same reference number are retained for corresponding features.

In this embodiment, the guidance apparatus comprises an antenna carrier 92 on which the RF antenna 18 is mounted. The antenna carrier is movably mounted and is arranged for linear movement, rotation and tilting. The controller 94 is arranged to generate a control signal 98 comprising instructions to cause the antenna carrier 92 to undergo at least one of a predetermined linear movement, a predetermined rotation, and a predetermined tilting movement. The RF signal can therefore be moved linearly relative to the surgical instrument set, may be rotated about the instrument set or may be tilted about the instrument set 14, or any combination of these, to cause a predetermined relative movement between the RF signal and the surgical instrument RFID tags.

A fourteenth embodiment provides surgical instrument radio frequency identification, RFID, tag reading apparatus 100 as shown in Figure 10. The apparatus 100 of this embodiment is similar to the apparatus 30 of Figure 2, with the following modifications. The same reference number are retained for corresponding features.

In this embodiment, a second RF antenna 102 is provided, generally opposite the RF antenna 18 and underneath the conveyor apparatus 32, such that the interrogation zone is located generally between the RF antennas. The second antenna is coupled to the RFID tag reader 16 and its RF signal 104 extends into the interrogation zone and partially overlaps with the RF signal 20 of the RF antenna 18.

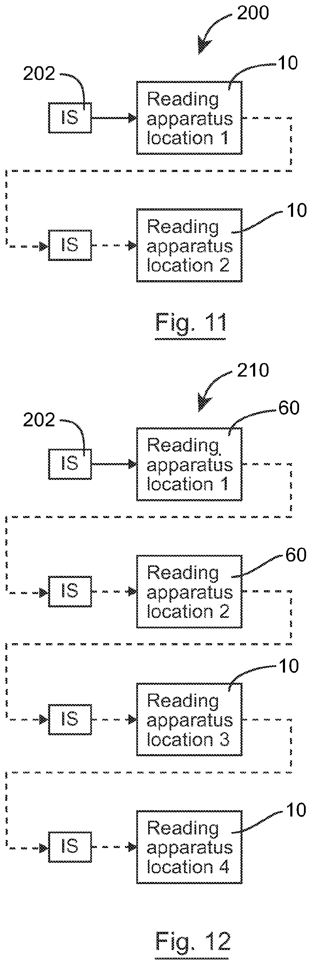

Referring to Figure 11, a fifteenth embodiment provides a surgical instrument tracking system 200. The system 200 comprises a surgical instrument set, IS, 202 and a plurality, two in this example, of surgical instrument RFID tag reading apparatus 10, as described above with reference to Figure 1. It will be appreciated that any of the above described surgical instrument RFID tag reading apparatus 30, 40, 50, 60, 70, 80, 90, 100 described with reference to Figures 2 to 10 may alternatively be used.

The instrument set 202 comprises a plurality of surgical instruments each comprising a respective RFID tag containing identification information for the surgical instrument.

Each surgical instrument RFID tag reading apparatus is arranged to receive the surgical instrument set at a respective location.

For example, the system 200 may be used within an operating theatre of a hospital. The surgical instrument RFID tag reading apparatus 10 provided at location 1 may be used to check that the instrument set IS 202 is complete before an operation is commenced, and the apparatus at location 2 may be used to check that the instrument set IS 202 is complete once the operation is completed. The surgical instruments can therefore be tracked over the course of an operation, to ensure that all of the surgical instruments required to perform the operation are present before starting the operation and to ensure that all of the surgical instruments are accounted for after the operation.

Alternatively, the system 200 may be used within the CSSD of a hospital. The surgical instrument RFID tag reading apparatus 10 provided at location 1 may be used to inspect the instrument set IS 202 before commencing the autoclave sterilisation and decontamination, and the apparatus at location 2 may be used to check that the instrument set IS 202 is complete once sterilisation and decontamination is completed. The surgical instruments can therefore be tracked over the sterilisation and decontamination process, to ensure that all of the surgical instruments are accounted for and the instrument set is complete and ready for use.

A sixteenth embodiment provides a surgical instrument tracking system 210 as shown in Figure 12. The system 210 of this embodiment is similar to the system 200 of the previous embodiment, with the following modifications. The same reference numbers are retained for corresponding features.

In this embodiment, the system 210 comprises two surgical instrument RFID tag reading apparatus 10, as described above with reference to Figure 1, and two surgical instrument RFID tag reading apparatus 60, as described above with reference to Figure 5.

This system 210 may be used within a hospital to track a surgical instrument set during the sterilisation and decontamination process and during use to perform an operation. The first two surgical instrument RFID tag reading apparatus 60 may be provided within the CSSD, as described above, and the second two surgical instrument RFID tag reading apparatus 10 may be provided in an operating theatre, as described above.

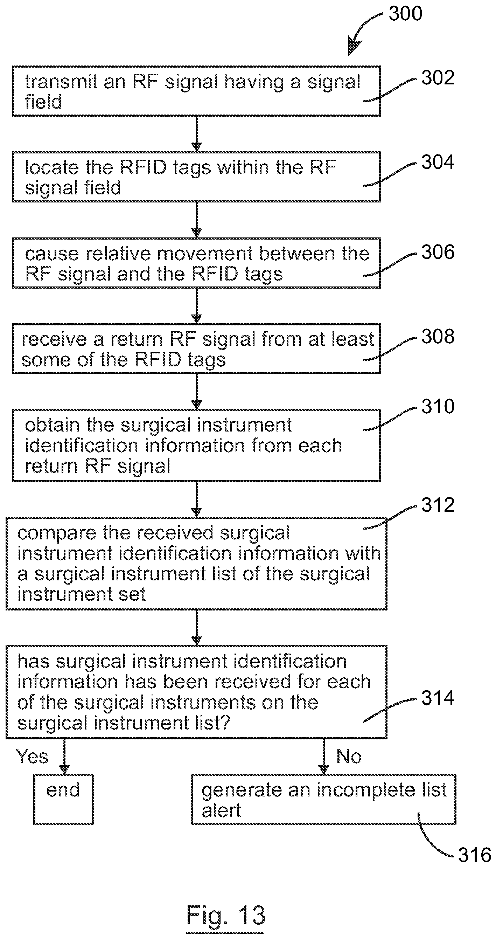

Referring to Figure 13, a seventeenth embodiment provides a method 300 of reading RFID tags on a plurality of surgical instruments of a surgical instrument set. Each RFID tag contains identification information for the respective surgical instrument.

The method 300 comprises the following steps:
a. transmitting an RF signal having a signal field 302;
b. locating the RFID tags within the RF signal field 304;
c. causing a predetermined relative movement between the RF signal and the RFID tags 306;
d. receiving a RF return signal from at least some of the RFID tags 308, each RF signal return containing the identification information of the respective RFID tag;
e. obtaining the surgical instrument identification information from each RF return signal 310;
f. comparing the received surgical instrument identification information with a surgical instrument list of the surgical instrument set 312; and
g. determining whether surgical instrument identification information has been received for each of the surgical instruments on the surgical instrument list 314, and if not, generating an incomplete list alert 316.

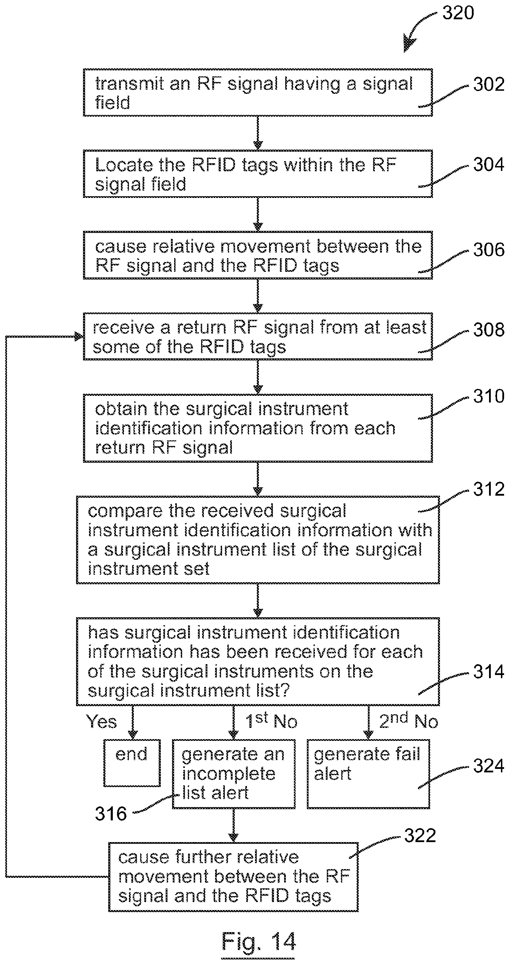

Referring to Figure 14, an eighteenth embodiment provides a method 320 of reading RFID tags on a plurality of surgical instruments of a surgical instrument set. The method 320 of this embodiment is similar to the method 300 of the previous embodiment, with the following modifications. The same reference numbers are retained for corresponding steps.

In this embodiment, the method 320 comprises, in response to an incomplete list alert 316, the following additional steps:
h. causing further predetermined relative movement between the RF signal and the RFID tags 322, the further predetermined relative movement being different to said predetermined relative movement; and
i. repeating steps a. 302 to g. 314.

If after repeating step g., surgical instrument identification information has still not been cumulatively received for all of the surgical instruments on the surgical instrument list, the method proceeds to generate a fail alert 324.

The predetermined relative movement performed in step c. 306 comprises one of linear movement in a first direction, rotation by a first angle, and tilting in a first plane and by a first angle. The further predetermined relative movement performed in step h. 322 comprises one of linear movement in a second direction, different to the first direction, rotation by a second angle different to the first angle, tilting in a second plane different to the first plane, tilting by a second angle different to the first angle, or a different one of linear movement in a first direction, rotation by a first angle, and tilting in a first plane and by a first angle.

In a nineteenth embodiment, also described with reference to Figure 14, in step c. 306 the predetermined relative movement is movement along a predetermined path at a first speed and in step h. 322 the further predetermined relative movement is movement along the predetermined path at a second speed, different to the first. Each linear movement may be at a constant speed or may follow a speed profile, in which the speed of movement is varied. The further predetermined movement may alternatively be along a different predetermined path.

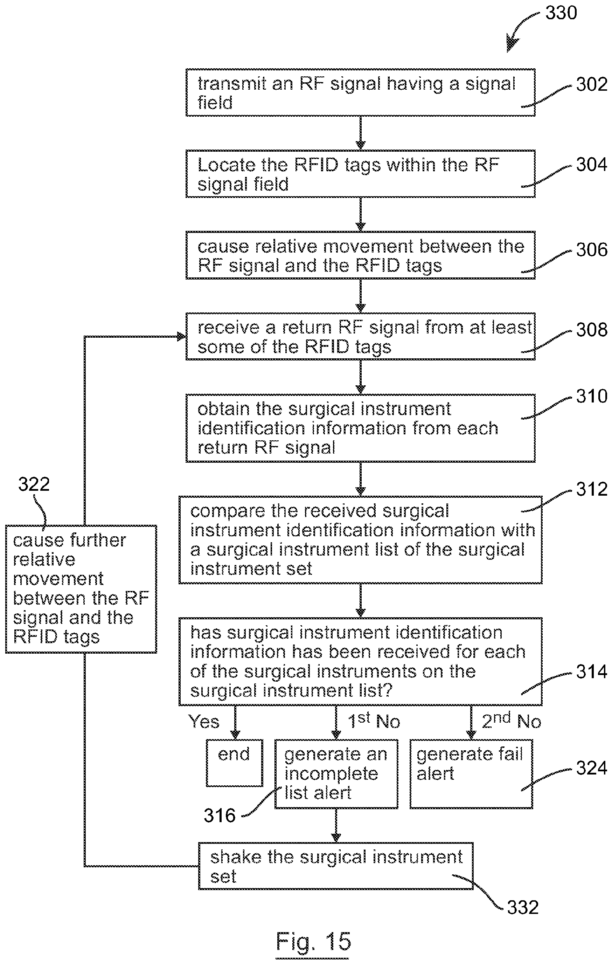

Referring to Figure 15, a twentieth embodiment provides a method 330 of reading RFID tags on a plurality of surgical instruments of a surgical instrument set. The method 330 of this embodiment is similar to the method 320 of the previous embodiment, with the following modifications. The same reference numbers are retained for corresponding steps.

In this embodiment, the method 330 additionally comprises shaking the surgical instrument set 332 such that the position of at least some of the surgical instruments is changed. The shaking is performed before the further relative movement 322.

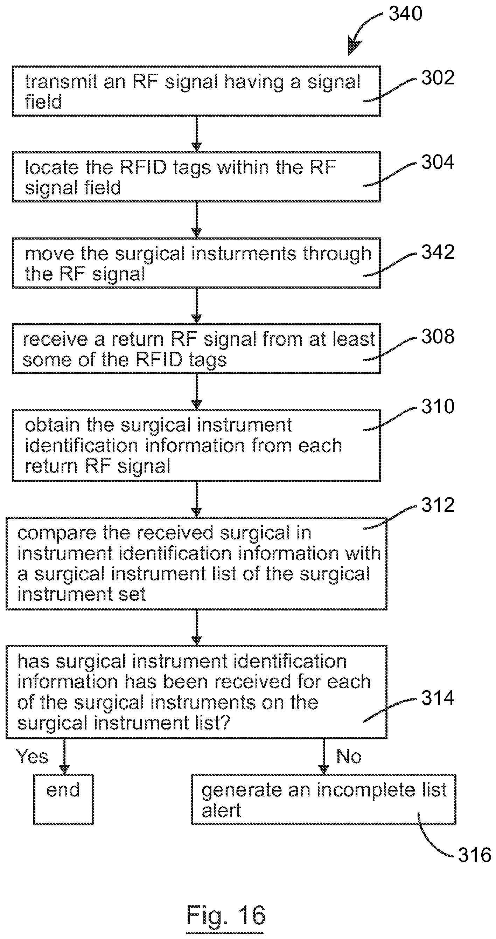

Referring to Figure 16, a twenty-first embodiment provides a method 340 of reading RFID tags on a plurality of surgical instruments of a surgical instrument set. The method 340 of this embodiment is similar to the method 300 described with reference to Figure 13, with the following modifications. The same reference numbers are retained for corresponding steps.

In this embodiment, the surgical instrument set, and thus the RFID tags on the surgical instruments, is moved through the RF signal 342.

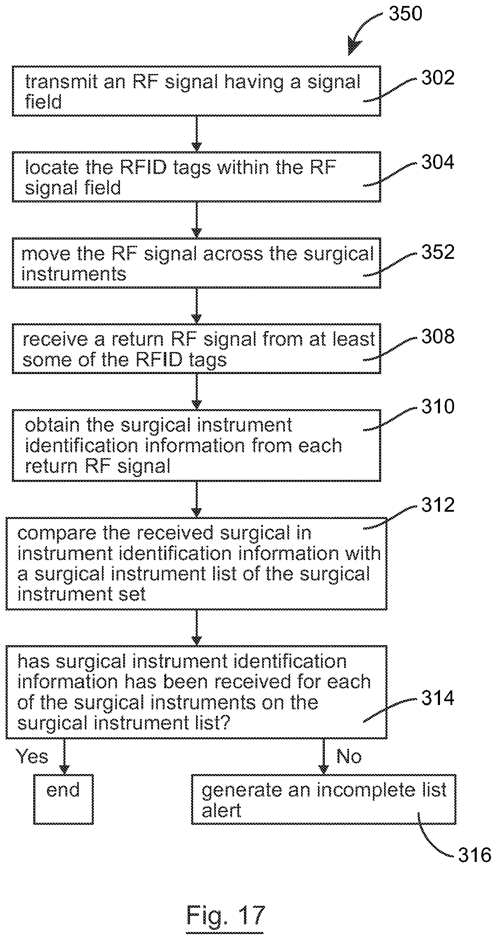

Referring to Figure 17, a twenty-second embodiment provides a method 350 of reading RFID tags on a plurality of surgical instruments of a surgical instrument set. The method 350 of this embodiment is similar to the method 300 described with reference to Figure 13, with the following modifications. The same reference numbers are retained for corresponding steps.

In this embodiment, the RF signal is moved across the surgical instruments, and thus across the RFID tags on the surgical instruments.

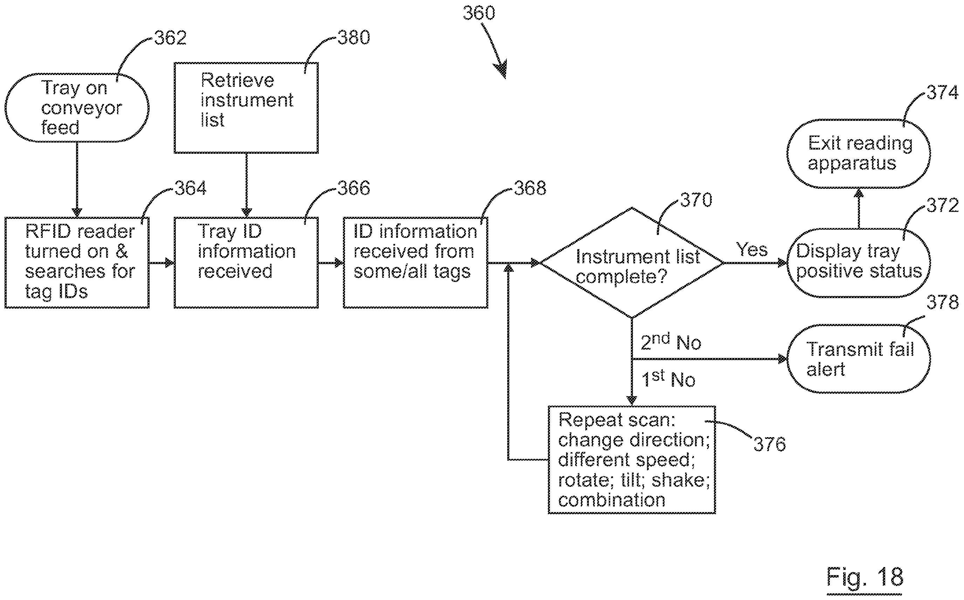

Referring to Figure 18, a twenty-third embodiment provides a method 360 of reading RFID tags on a plurality of surgical instruments of a surgical instrument set.

The method 360 is implemented using the apparatus 60 shown in Figure 5. The instrument set 14 of this embodiment additionally has an RFID tag on the instrument tray.

The method comprises:
placing the instrument tray containing the surgical instruments on the conveyor apparatus 362;
turning on the RFID reader and the RF antenna to transmit an RF signal and searching for identification, ID, information for the RFID tags on the surgical instruments 364;
receiving ID information for the tray 366;
retrieving the instrument list for the tray 380;
receiving surgical instrument ID information from at least some of the RFID tags on the surgical instruments 368;
comparing the received surgical instrument ID information with the surgical instrument list and determining whether ID information has been received for each of the surgical instruments on the list 370;
if ID information has been received for each of the surgical instruments on the list, displaying a 'tray positive' status 372 and exiting the tray from the apparatus 374; and
if ID information has not been received for each of the surgical instruments on the list, causing further relative movement between the RF signal and the RFID tags, by repeating the scan by at least one of: moving the conveyor apparatus in the opposite direction; moving the conveyor apparatus at a different speed; rotating the interrogation platform; tilting the interrogation platform; and shaking the interrogation platform or moving the conveyor apparatus backwards and forwards to shake the instrument tray;
comparing all the received surgical instrument ID information with the surgical instrument list and determining whether ID information has been received for each of the surgical instruments on the list 370; and
if ID information has been received for each of the surgical instruments on the list, displaying a 'tray positive' status 372 and exiting the tray from the apparatus 374; and
if ID information has still not been received for each of the surgical instruments on the list, transmitting a fail alert 378.

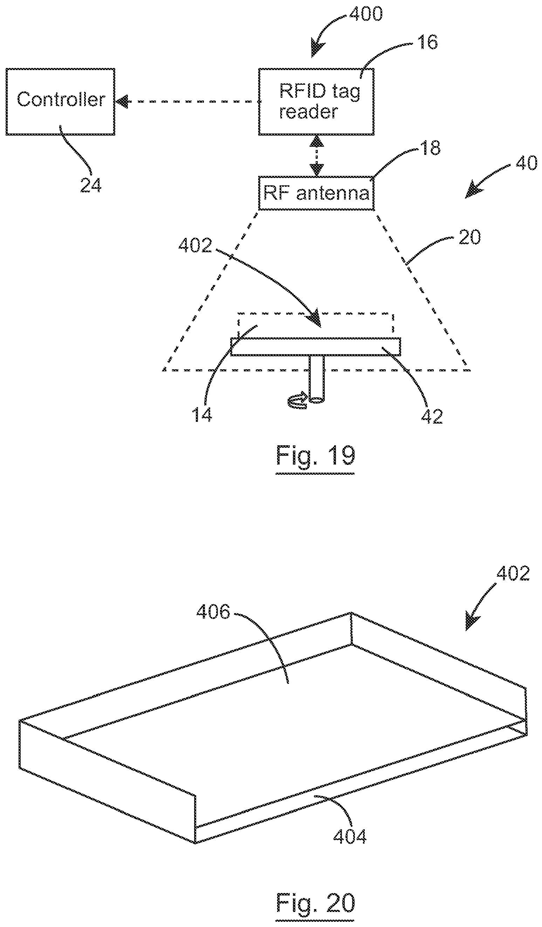

Referring to Figures 19 and 20, a twenty-fourth embodiment provides a surgical instrument RFID tag reading system 400 comprising a surgical instrument RFID tag reading apparatus 10 and an instrument carrier, in the form of an instrument tray 402. The instrument tray comprises a base 404 and an instrument platform 406. The instrument platform is mounted within the instrument tray raised above the base. The base is reflective to RF signals and the instrument platform is at least partially transparent to RF signals.

It will be appreciated that any of the above described surgical instrument RFID tag reading apparatus 10, 30, 50, 60, 70, 80, 90, 100 described with reference to Figures 2 to 10 may alternatively be used.

## Claims

1. Surgical instrument radio frequency identification, RFID, tag reading apparatus (10, 30, 40, 50, 60, 70, 80, 90) comprising:
an interrogation zone (12) arranged to receive a surgical instrument set (14) comprising a plurality of surgical instruments, each surgical instrument comprising a respective RFID tag containing identification information for the surgical instrument;
an RFID tag reader (16);
a radio frequency, RF, antenna (18) coupled to the RFID tag reader and arranged to transmit an RF signal (20) having a signal field at least part of which extends within the interrogation zone and arranged to receive RF return signals from at least some of the RFID tags, each RF return signal containing the identification information of the respective RFID tag;
guidance apparatus (22, 32, 42, 52, 62, 64, 72, 82) arranged to cause a predetermined relative movement between the RFID tags on the surgical instruments and the RF signal; and
a controller (24) arranged to:
a. receive the surgical instrument identification information from the RFID tag reader for each RFID tag from which an RF return signal is received;
b. compare the received surgical instrument identification information with a surgical instrument list of the surgical instrument set;
c. determine whether surgical instrument identification information has been received for each of the surgical instruments on the surgical instrument list, and if not: generate an incomplete list alert,
and **characterised in that** the guidance apparatus is arranged to cause a predetermined rotational movement between the RF antenna (18) and the surgical instrument set (14) and
the controller is further arranged to, in response to an incomplete list alert:
d. transmit a control signal (68) to the guidance apparatus comprising instructions arranged to cause a predetermined rotational relative movement between the RFID tags on the surgical instruments and the RF antenna (18), the predetermined rotational relative movement being different to said predetermined relative movement; and
e. repeat a. to c.

2. Surgical instrument RFID tag reading apparatus as claimed in claim 1, wherein the controller is arranged to generate and transmit a control signal (68) to the guidance apparatus comprising instructions to cause the guidance apparatus to guide movement of the surgical instrument set along a predetermined path at a speed which varies along said path, and wherein the speed varies according to a predetermined speed profile.

3. Surgical instrument RFID tag reading apparatus as claimed in claim 1 or claim 2, wherein the guidance apparatus (42, 52, 62, 72, 82) is movably mounted and is arranged to perform at least one of rotation and tilting, such that a predetermined rotational or tilting relative movement is caused between the RF antenna (18) and the surgical instrument set, and shaking, such that the position of at least some of the surgical instruments is changed.

4. Surgical instrument RFID tag reading apparatus as claimed in any of claims 1 to 3, wherein the guidance apparatus (42) comprises weighing apparatus and the controller is further arranged to:
receive a weight of the surgical instrument set from the weighing apparatus;
compare the weight with a reference weight associated with the surgical instrument list; and
determine whether the received weight is substantially equal to the reference weight, and if not, generate an incomplete list alert at c.

5. Surgical instrument RFID tag reading apparatus as claimed in any of claims 1 to 4, wherein the guidance apparatus comprises an antenna carrier (92) on which the RF antenna is mounted, the antenna carrier being movably mounted and arranged to undergo at least one of a predetermined linear movement, rotation and tilting such that at least one of a predetermined linear, rotational and tilting relative movement is caused between the RF antenna (18) and the surgical instrument set.

6. A surgical instrument RFID tag reading system (400) comprising:
surgical instrument RFID tag reading apparatus (10, 30, 40, 50, 60, 70, 80, 90) as claimed in any preceding claim; and
an instrument carrier (402) comprising: a base (404) which is reflective to RF signals; and an instrument platform (406) which is at least partially transparent to RF signals, the instrument platform being mounted within the instrument carrier raised above the base.

7. A surgical instrument tracking system (200, 210) comprising:
a surgical instrument set (202) comprising a plurality of surgical instruments each comprising a respective RFID tag containing identification information for the surgical instrument; and
a plurality of surgical instrument RFID tag reading apparatus (10, 30, 40, 50, 60, 70, 80, 90) as claimed in any of claims 1 to 5, each apparatus arranged to receive the surgical instrument set.

8. A method (300) of reading RFID tags on a plurality of surgical instruments of a surgical instrument set, each RFID tag containing identification information for the respective surgical instrument, the method comprising steps:
a. transmitting an RF signal having a signal field (302);
b. locating the RFID tags within the RF signal field (304);
c. causing a predetermined relative movement between the RF signal field and the RFID tags (306);
d. receiving a RF return signal from at least some of the RFID tags (308), each RF return signal containing the identification information of the respective RFID tag;
e. obtaining the surgical instrument identification information from each RF return signal (310);
f. comparing the received surgical instrument identification information with a surgical instrument list of the surgical instrument set (312);
g. determining whether surgical instrument identification information has been received for each of the surgical instruments on the surgical instrument list (314), and if not: generating an incomplete list alert (316) and
**characterised by** further comprising, in response to an incomplete list alert, steps:
h. causing a predetermined rotational relative movement between the RF signal field and the surgical instrument set, the rotational relative movement being different to said predetermined relative movement; and
i. repeating steps a. to g.

9. A method as claimed in claim 8, wherein the predetermined relative movement comprises movement along a predetermined path at a speed which varies along said path, and wherein the speed is varied along said path according to a predetermined speed profile.

10. A method as claimed in claim 8 or claim 9, wherein in step c. the predetermined relative movement comprises one of linear movement in a first direction, rotation by a first angle, and tilting in a first plane and by a first angle.

11. A method as claimed in claim 10, wherein in step h. the further relative movement is one of movement in a second direction, different to the first direction, rotation by a second angle different to the first angle, tilting in a second plane different to the first plane, tilting by a second angle different to the first angle, or a different one of linear movement in a first direction, rotation by a first angle, and tilting in a first plane and by a first angle.

12. A method as claimed in any of claims 8 to 11, wherein step h. additionally comprises shaking the surgical instrument set prior to the further relative movement, such that the position of at least some of the surgical instruments is changed.

13. A method as claimed in any of claims 8 to 12, wherein the relative movement between the RF signal field and the RFID tags comprises movement of the surgical instruments through the RF signal field.

14. A method as claimed in any of claims 8 to 13, wherein the relative movement between the RF signal field and the RFID tags comprises movement of the RF signal field across the surgical instruments.

## Patentansprüche

1. Radiofrequenzidentifikation für chirurgische Instrumente, RFID, Tag-Lesegerät (10, 30, 40, 50, 60, 70, 80, 90) für chirurgische Instrumente, umfassend:
eine Abfragezone (12), die so angeordnet ist, dass sie einen chirurgischen Instrumentensatz (14) aufnimmt, der eine Vielzahl von chirurgischen Instrumenten umfasst, wobei jedes chirurgische Instrument ein entsprechendes RFID-Tag umfasst, das Identifikationsinformationen für das chirurgische Instrument enthält;
einen RFID-Tag-Leser (16);
eine Hochfrequenz-HF-Antenne (18), die mit dem RFID-Tag-Leser gekoppelt ist und zum Übertragen eines HF-Signals (20) mit einem Signalfeld angeordnet ist, das sich mindestens teilweise innerhalb der Abfragezone erstreckt, und zum Empfangen von HF-Rückmeldesignalen von mindestens einigen der RFID-Tags angeordnet ist, wobei jedes HF-Rückmeldesignal die Identifikationsinformationen des jeweiligen RFID-Tags enthält;
eine Führungsvorrichtung (22, 32, 42, 52, 62, 64, 72, 82), die so angeordnet ist, dass sie eine vorgegebene Relativbewegung zwischen den RFID-Tags an den chirurgischen Instrumenten und dem HF-Signal bewirkt; und
eine Steuerung (24), die angeordnet ist zum:
a. Empfangen der Identifikationsinformationen des chirurgischen Instruments von dem RFID-Tag-Leser für jedes RFID-Tag, von dem ein HF-Rückmeldesignal empfangen wird;
b. Vergleichen der empfangenen Identifikationsinformationen des chirurgischen Instruments mit einer Liste der chirurgischen Instrumente des chirurgischen Instrumentensatzes;
c. Bestimmen, ob für jedes der chirurgischen Instrumente in der Liste der chirurgischen Instrumente Identifikationsinformationen des chirurgischen Instruments empfangen wurden, und wenn nicht: Generieren eines Alarms "Liste unvollständig", und **dadurch gekennzeichnet, dass** die Führungsvorrichtung angeordnet ist, um eine vorgegebene Rotationsbewegung zwischen der HF-Antenne (18) und dem chirurgischen Instrumentensatz (14) zu bewirken und die Steuerung, als Reaktion auf einen Alarm "Liste unvollständig", ferner angeordnet ist zum:
d. Übertragen eines Steuersignals (68) an die Führungsvorrichtung, umfassend Anweisungen, die dazu angeordnet sind, eine vorgegebene Rotations-Relativbewegung zwischen den RFID-Tags an den chirurgischen Instrumenten und der HF-Antenne (18) zu bewirken, wobei sich die vorgegebene Rotations-Relativbewegung von der vorgegebenen Relativbewegung unterscheidet; und
e. Wiederholen von a. bis c.

2. RFID-Tag-Lesevorrichtung für chirurgische Instrumente nach Anspruch 1, wobei die Steuerung so angeordnet ist, dass sie ein Steuersignal (68) generiert und an die Führungsvorrichtung überträgt, das Anweisungen umfasst, um zu bewirken, dass die Führungsvorrichtung die Bewegung des chirurgischen Instrumentensatzes entlang eines vorgegebenen Pfades mit einer Geschwindigkeit führt, die entlang des Pfades variiert, und wobei die Geschwindigkeit gemäß einem vorgegebenen Geschwindigkeitsprofil variiert.

3. RFID-Tag-Lesevorrichtung für chirurgische Instrumente nach Anspruch 1 oder Anspruch 2, wobei die Führungsvorrichtung (42, 52, 62, 72, 82) beweglich montiert ist und so angeordnet ist, dass sie mindestens eines von Rotation und Kippen ausführt, sodass eine vorgegebene Rotations- oder Kipp-Relativbewegung zwischen der HF-Antenne (18) und dem chirurgischen Instrumentensatz, und Schütteln bewirkt wird, sodass die Position von mindestens einigen der chirurgischen Instrumente verändert wird.

4. RFID-Tag-Lesevorrichtung für chirurgische Instrumente nach einem der Ansprüche 1 bis 3, wobei die Führungsvorrichtung (42) eine Wiegevorrichtung umfasst und die Steuerung ferner angeordnet ist zum:
Empfangen eines Gewichts des chirurgischen Instrumentensatzes von der Wiegevorrichtung; Vergleichen des Gewichts mit einem Referenzgewicht, das der Liste der chirurgischen Instrumente zugeordnet ist, und
Bestimmen, ob das empfangene Gewicht im Wesentlichen gleich dem Referenzgewicht ist, und wenn nicht, Generieren eines Alarms "Liste unvollständig" bei c.

5. RFID-Tag-Lesevorrichtung für chirurgische Instrumente nach einem der Ansprüche 1 bis 4, wobei die Führungsvorrichtung einen Antennenträger (92) umfasst, auf dem die HF-Antenne montiert ist, wobei der Antennenträger beweglich montiert und so angeordnet ist, dass er mindestens einem von einer vorgegebenen linearen Bewegung, Rotation und Kippen unterzogen wird, sodass mindestens eines von einer vorgegebenen linearen, Rotations- und Kipp-Relativbewegung zwischen der HF-Antenne (18) und dem chirurgischen Instrumentensatz bewirkt wird.

6. RFID-Tag-Lesesystem für chirurgische Instrumente (400), umfassend:
RFID-Tag-Lesevorrichtung für chirurgische Instrumente (10, 30, 40, 50, 60, 70, 80, 90) nach einem der vorhergehenden Ansprüche; und
einen Instrumententräger (402), umfassend: eine Basis (404), die HF-Signale reflektiert; und
eine Instrumentenplattform (406), die mindestens teilweise für HF-Signale transparent ist, wobei die Instrumentenplattform in dem über der Basis erhöhten Instrumententräger montiert ist.

7. Verfolgungssystem für chirurgische Instrumente (200, 210), umfassend:
einen chirurgischen Instrumentensatz (202), der eine Vielzahl von chirurgischen Instrumenten umfasst, die jeweils ein jeweiliges RFID-Tag umfassen, das Identifikationsinformationen für das chirurgische Instrument enthält; und
eine Vielzahl von RFID-Tag-Lesevorrichtungen für chirurgische Instrumente (10, 30, 40, 50, 60, 70, 80, 90) nach einem der Ansprüche 1 bis 5, wobei jede Vorrichtung so angeordnet ist, dass sie den chirurgischen Instrumentensatz aufnimmt.

8. Verfahren (300) zum Lesen von RFID-Tags auf einer Vielzahl von chirurgischen Instrumenten eines chirurgischen Instrumentensatzes, wobei jedes RFID-Tag Identifikationsinformation für das jeweilige chirurgische Instrument enthält, wobei das Verfahren die folgenden Schritte umfasst:
a. Übertragen eines HF-Signals mit einem Signalfeld (302);
b. Lokalisieren der RFID-Tags innerhalb des HF-Signalfelds (304);
c. Bewirken einer vorgegebenen Relativbewegung zwischen dem HF-Signalfeld und den RFID-Tags (306);
d. Empfangen eines HF-Rückmeldesignals von mindestens einigen der RFID-Tags (308), wobei jedes RF-Rückmeldesignal die Identifikationsinformationen des jeweiligen RFID-Tags enthält;
e. Erhalten der Identifikationsinformationen des chirurgischen Instruments von jedem HF-Rückmeldesignal (310);
f. Vergleichen der empfangenen Identifikationsinformationen des chirurgischen Instruments mit einer Liste der chirurgischen Instrumente des chirurgischen Instrumentensatzes (312);
g. Bestimmen, ob für jedes der chirurgischen Instrumente auf der Liste der chirurgischen Instrumente (314) Identifikationsinformationen des chirurgischen Instruments empfangen wurden, und falls nicht: Generieren eines Alarms "Liste unvollständig" (316), und **dadurch gekennzeichnet, dass** es ferner, als Reaktion auf einen Alarm "Liste unvollständig", die folgenden Schritte umfasst:
h. Bewirken einer vorgegebenen Rotations-Relativbewegung zwischen dem HF-Signalfeld und dem chirurgischen Instrumentensatz, wobei sich die Rotations-Relativbewegung von der vorgegebenen Relativbewegung unterscheidet; und
i. Wiederholen der Schritte a. bis g.

9. Verfahren nach Anspruch 8, bei dem die vorgegebene Relativbewegung eine Bewegung entlang eines vorgegebenen Pfades mit einer Geschwindigkeit, die entlang des Pfades variiert, umfasst, und wobei die Geschwindigkeit entlang des Pfades entsprechend einem vorgegebenen Geschwindigkeitsprofil variiert wird.

10. Verfahren nach Anspruch 8 oder Anspruch 9, wobei in Schritt c. die vorgegebene Relativbewegung eines von einer linearen Bewegung in einer ersten Richtung, Rotation um einen ersten Winkel, und Kippen in einer ersten Ebene und um einen ersten Winkel, umfasst.

11. Verfahren nach Anspruch 10, wobei in Schritt h. die weitere Relativbewegung eines ist von einer Bewegung in einer zweiten Richtung, die sich von der ersten Richtung unterscheidet, Rotation um einen zweiten Winkel, der sich von dem ersten Winkel unterscheidet, Kippen in einer zweiten Ebene, die sich von der ersten Ebene unterscheidet, Kippen um einen zweiten Winkel, der sich von dem ersten Winkel unterscheidet, oder ein anderes ist von einer linearen Bewegung in einer ersten Richtung, Rotation um einen ersten Winkel, und Kippen in einer ersten Ebene und um einen ersten Winkel.

12. Verfahren nach einem der Ansprüche 8 bis 11, bei dem Schritt h. zusätzlich das Schütteln des chirurgischen Instrumentensatzes vor der weiteren Relativbewegung umfasst, sodass die Position von mindestens einigen der chirurgischen Instrumente verändert wird.

13. Verfahren nach einem der Ansprüche 8 bis 12, wobei die Relativbewegung zwischen dem HF-Signalfeld und den RFID-Tags die Bewegung der chirurgischen Instrumente durch das HF-Signalfeld umfasst.

14. Verfahren nach einem der Ansprüche 8 bis 13, wobei die Relativbewegung zwischen dem HF-Signalfeld und den RFID-Tags die Bewegung des HF-Signalfelds über die chirurgischen Instrumente umfasst.

## Revendications

1. Appareil de lecture d'étiquettes d'identification par radiofréquence, RFID, pour un instrument chirurgical, (10, 30, 40, 50, 60, 70, 80, 90) comprenant :
une zone d'interrogation (12) conçue pour recevoir un ensemble d'instruments chirurgicaux (14) comprenant une pluralité d'instruments chirurgicaux, chaque instrument chirurgical comprenant une étiquette RFID respective contenant des informations d'identification pour l'instrument chirurgical ;
un lecteur d'étiquettes RFID (16) ;
une antenne radiofréquence RF (18) couplée au lecteur d'étiquettes RFID et conçue pour transmettre un signal RF (20) ayant un champ de signal dont au moins une partie s'étend à l'intérieur de la zone d'interrogation et conçue pour recevoir des signaux de retour RF d'au moins certaines des étiquettes RFID, chaque signal de retour RF contenant les informations d'identification de l'étiquette RFID respective ;
un appareil de guidage (22, 32, 42, 52, 62, 64, 72, 82) conçu pour entraîner un mouvement relatif prédéterminé entre les étiquettes RFID sur les instruments chirurgicaux et le signal RF ; et
un dispositif de commande (24) conçu pour :
a. recevoir les informations d'identification de l'instrument chirurgical à partir du lecteur d'étiquettes RFID pour chaque étiquette RFID à partir de laquelle un signal de retour RF est reçu ;
b. comparer les informations d'identification de l'instrument chirurgical reçues à une liste d'instruments chirurgicaux de l'ensemble d'instruments chirurgicaux ;
c. déterminer si des informations d'identification de l'instrument chirurgical ont été reçues pour chacun des instruments chirurgicaux sur la liste d'instruments chirurgicaux, et sinon : générer une alerte de liste incomplète, et **caractérisé en ce que** l'appareil de guidage est conçu pour entraîner un mouvement de rotation prédéterminé entre l'antenne RF (18) et l'ensemble d'instruments chirurgicaux (14) et le dispositif de commande est en outre conçu pour, en réponse à une alerte de liste incomplète :
d. transmettre un signal de commande (68) à l'appareil de guidage comprenant des instructions conçues pour entraîner un mouvement relatif de rotation prédéterminé entre les étiquettes RFID sur les instruments chirurgicaux et l'antenne RF (18), le mouvement relatif de rotation prédéterminé étant différent dudit mouvement relatif prédéterminé ; et
e. répéter a. à c.

2. Appareil de lecture d'étiquettes RFID pour un instrument chirurgical selon la revendication 1, dans lequel le dispositif de commande est conçu pour générer et transmettre un signal de commande (68) à l'appareil de guidage comprenant des instructions pour entraîner l'appareil de guidage à guider un mouvement de l'ensemble d'instruments chirurgicaux le long d'un trajet prédéterminé à une vitesse qui varie le long dudit trajet, et dans lequel la vitesse varie selon un profil de vitesse prédéterminé.

3. Appareil de lecture d'étiquettes RFID pour un instrument chirurgical selon la revendication 1 ou la revendication 2, dans lequel l'appareil de guidage (42, 52, 62, 72, 82) est monté de façon mobile et est conçu pour effectuer au moins l'un d'une rotation et une inclinaison, de sorte qu'un mouvement relatif prédéterminé en rotation ou en inclinaison est entraîné entre l'antenne RF (18) et l'ensemble d'instruments chirurgicaux, et des agitations, de sorte que la position d'au moins certains parmi les instruments chirurgicaux est modifiée.

4. Appareil de lecture d'étiquettes RFID pour un instrument chirurgical selon l'une quelconque des revendications 1 à 3, dans lequel l'appareil de guidage (42) comprend un appareil de pesage et le dispositif de commande est en outre conçu pour :
recevoir un poids de l'ensemble d'instruments chirurgicaux de l'appareil de pesage ; comparer le poids à un poids de référence associé à la liste d'instruments chirurgicaux ; et
déterminer si le poids reçu est sensiblement égal au poids de référence, et sinon, générer une alerte de liste incomplète au niveau de c.

5. Appareil de lecture d'étiquettes RFID pour un instrument chirurgical selon l'une quelconque des revendications 1 à 4, dans lequel l'appareil de guidage comprend un porte-antenne (92) sur lequel l'antenne RF est montée, le porte-antenne étant monté de façon mobile et conçu pour subir un mouvement linéaire prédéterminé, et/ou une rotation et/ou une inclinaison tel qu'un mouvement relatif linéaire, et/ou rotatif et/ou d'inclinaison prédéterminé soit entraîné entre l'antenne RF (18) et l'ensemble d'instruments chirurgicaux.

6. Système de lecture d'étiquettes RFID pour un instrument chirurgical (400) comprenant :
un appareil de lecture d'étiquettes RFID pour un instrument chirurgical (10, 30, 40, 50, 60, 70, 80, 90) selon l'une quelconque des revendications précédentes ; et
un porte-instrument (402) comprenant : une base (404) qui réfléchit les signaux RF ; et
une plate-forme d'instrument (406) qui est au moins partiellement transparente aux signaux RF, la plate-forme d'instrument étant montée à l'intérieur du porte-instrument élevé au-dessus de la base.

7. Système de poursuite d'un instrument chirurgical (200, 210) comprenant :
un ensemble d'instruments chirurgicaux (202) comprenant une pluralité d'instruments chirurgicaux comprenant chacun une étiquette RFID respective contenant des informations d'identification pour l'instrument chirurgical ; et
une pluralité d'appareils de lecture d'étiquettes RFID pour un instrument chirurgical (10, 30, 40, 50, 60, 70, 80, 90) selon l'une quelconque des revendications 1 à 5, chaque appareil étant conçu pour recevoir l'ensemble d'instruments chirurgicaux.

8. Procédé (300) de lecture d'étiquettes RFID sur une pluralité d'instruments chirurgicaux d'un ensemble d'instruments chirurgicaux, chaque étiquette RFID contenant des informations d'identification pour l'instrument chirurgical respectif, le procédé comprenant les étapes consistant à :
a. transmettre un signal RF ayant un champ de signal (302) ;
b. localiser les étiquettes RFID à l'intérieur du champ de signal RF (304) ;
c. entraîner un mouvement relatif prédéterminé entre le champ de signal RF et les étiquettes RFID (306) ;
d. recevoir un signal de retour RF à partir d'au moins certaines des étiquettes RFID (308), chaque signal de retour RF contenant les informations d'identification de l'étiquette RFID respective ;
e. obtenir les informations d'identification de l'instrument chirurgical à partir de chaque signal de retour RF (310) ;
f. comparer les informations d'identification de l'instrument chirurgical reçues à une liste d'instruments chirurgicaux de l'ensemble d'instruments chirurgicaux (312) ;
g. déterminer si des informations d'identification de l'instrument chirurgical ont été reçues pour chacun des instruments chirurgicaux sur la liste d'instruments chirurgicaux (314), et sinon : générer une alerte de liste incomplète (316) et **caractérisé en ce qu'**il comprend en outre, en réponse à une alerte de liste incomplète, les étapes consistant à :
h. entraîner un mouvement relatif de rotation prédéterminé entre le champ de signal RF et l'ensemble d'instruments chirurgicaux, le mouvement relatif de rotation étant différent dudit mouvement relatif prédéterminé ; et
i. répéter les étapes a. à g.

9. Procédé selon la revendication 8, dans lequel le mouvement relatif prédéterminé comprend un mouvement le long d'un chemin prédéterminé à une vitesse qui varie le long dudit chemin, et dans lequel la vitesse varie le long dudit chemin selon un profil de vitesse prédéterminé.

10. Procédé selon la revendication 8 ou la revendication 9, dans lequel à l'étape c. le mouvement relatif prédéterminé comprend l'un quelconque parmi un mouvement linéaire dans une première direction, une rotation selon un premier angle et une inclinaison dans un premier plan et selon un premier angle.

11. Procédé selon la revendication 10, dans lequel à l'étape h. le mouvement relatif supplémentaire est l'un quelconque parmi un mouvement dans une seconde direction, différente de la première direction, une rotation d'un second angle différent du premier angle, une inclinaison dans un second plan différent du premier plan, une inclinaison d'un second angle différent du premier angle, ou un mouvement différent parmi un mouvement linéaire dans une première direction, une rotation par un premier angle, et une inclinaison dans un premier plan et par un premier angle.

12. Procédé selon l'une quelconque des revendications 8 à 11, dans lequel l'étape h. comprend également l'agitation de l'ensemble d'instruments chirurgicaux avant le mouvement relatif supplémentaire, de sorte que la position d'au moins certains des instruments chirurgicaux est modifiée.

13. Procédé selon l'une quelconque des revendications 8 à 12, dans lequel le mouvement relatif entre le champ de signal RF et les étiquettes RFID comprend un mouvement des instruments chirurgicaux à travers le champ de signal RF.

14. Procédé selon l'une quelconque des revendications 8 à 13, dans lequel le mouvement relatif entre le champ de signal RF et les étiquettes RFID comprend un mouvement du champ de signal RF à travers les instruments chirurgicaux.
